# EUROPEAN PATENT SPECIFICATION

(11) **EP 1 594 531 B1**
(45) Date of publication and mention of the grant of the patent: **18.04.2012**
(21) Application number: 03815916.6
(22) Date of filing: 05.12.2003
(51) Int. Cl.: A61K 38/55, A61K 31/44, A61K 31/40

(54) **Storage-stable and bio-stable formulations of ACE inhibitors, and methods for preparation thereof**
Lagerstabile und biostabile ACE-Hemmer-Formulierungen und Herstellungsverfahren dafür
Formulations stables au stockage et biologiquement stables d'inhibiteurs des ACE, et procedes d'elaboration correspondants

(30) Priority: 12.02.2003 US 364970; 04.12.2003 US 727805
(43) Date of publication of application: 16.11.2005
(73) Proprietor: Sigmapharm, Inc., Newtown, PA 18940 (US)
(72) Inventor: SPIREAS, Spiridon, Newtown, PA 18940 (US)
(74) Representative: Gregory, Robert John H.
(86) International application number: PCT/US2003/040132
(87) International publication number: WO 2004/071526

(56) References cited:
- EP-A2- 0 425 892
- WO-A1-01/15724
- WO-A2-02/15891

## Description

### FIELD OF THE INVENTION

The present invention relates to storage-stable and bio-stable formulations of ACE inhibitors and similar drugs, especially enalapril maleate and quinapril hydrochloride. The present invention also relates to time and cost efficient methods of acceptable manufacturing characteristics for the preparation and industrial-scale production of storage-stable and bio-stable formulations of ACE inhibitors.

### BACKGROUND OF THE INVENTION

ACE inhibitors, or inhibitors of Angiotensin Converting Enzymes, are drugs useful in the treatment of cardiovascular disorders, especially hypertension. However, it has been widely observed that ACE inhibitors are susceptible to breakdown, especially due to hydrolytic degradation and/or cyclization between the time of manufacture and the time of desired usage. Breakdown of ACE inhibitors has been found to occur both in solid and in liquid states. Therefore, such breakdown can occur not only during the storage of solid dosage forms containing ACE inhibitors but also during the dissolution of ACE inhibitor products in the aqueous contents of the gastrointestinal tract. As breakdown of ACE inhibitor increases during storage, the concentration of available, functional ACE inhibitor decreases. Furthermore, when an ACE inhibitor product is administered orally, the ACE inhibitor hydrolyzes at various levels in the watery environment of the gastrointestinal tract producing moieties that can not be readily absorbed from the gastrointestinal membranes, thereby possibly creating unnecessary therapeutic variability and inequivalence. Also, at least some of the degradation products of such breakdown are believed to be deleterious. Accordingly, such breakdown of the ACE inhibitor during the storage and dissolution of the ACE inhibitor product is to be avoided.

ACE inhibitors include, but are not limited to, enalapril maleate and similar salts; quinapril hydrochloride and similar salts; benazepril hydrochloride and similar salts; moexipril hydrochloride and similar salts; lisonopril hydrochloride and similar salts; ramipril hydrochloride and similar salts; and indopril hydrochloride and similar salts. Typical breakdown products of ACE inhibitors include, but are not limited to, enalaprilat and/or enalaprildiketopiperazine (DKP) for enalapril species, quinaprilat and/or quinapril-DKP for quinapril drugs, and other breakdown products well-known to those of skill in the art.

Methods for the formulation of enalapril maleate, an ACE inhibitor, into stable solid dosage forms have been previously described. For example, Merslavic et al., in U.S. Patent 5,350,582, describe the formulation of enalapril sodium through the suspension of enalapril maleate in water and subsequent addition of certain metal compounds. Full conversion to enalapril sodium is said to be indicated by a final "clear" solution. However, the suspension of enalapril maleate in water is extremely time-consuming due to the low wetability of enalapril maleate. Consequently, the residence time of the drug in the water is high. A high residence time is believed to facilitate significant hydrolysis of the product and lead to a drop in drug purity. Further, following the procedures described by Merslavic et al., high unit dose weights of lactose and starch are required.

Sherman et al., in U.S. Patents 5,690,962 and 5,573,780, have described methods for the formulation of enalapril sodium. Instead of dispersing enalapril maleate in water, Sherman et al. describe "dry-blending" the enalapril maleate with an alkaline sodium powder and another powder excipient such as lactose. This "blend" is then granulated with water to initiate the acid-base conversion of enalapril maleate to enalapril sodium. Sherman et al. discuss a process which avoids converting the enalapril maleate to a clear solution of enalapril sodium and maleic acid sodium salt in water. Unlike the process described by Merslavic et al., Sherman provides no easily determinable endpoint of complete conversion of enalapril maleate to enalapril sodium. Therefore, it is likely that significant batch-to-batch variations in purity of the product, i.e., amount of enalapril sodium, will exist in large scale production scenarios. Additionally, the Sherman et al. process may involve a time-consuming conversion of enalapril maleate to enalapril sodium, such that the product is vulnerable to breakdown and a drop in drug purity.

Sherman et al., in U.S. Patent 5,562,921, also describe the manufacture of enalapril maleate formulations with improved resistance to decomposition. These formulations are said to be more resistant to decomposition due to restrictions in the excipients used in the process. However, the excipients set forth by Sherman as offering improved resistance to decomposition may lead to formulations which lack sufficient hardness, an important quality in pharmaceutical formulations.

Harris et al., in U.S. Patent 4,743,450, describe the use of stabilizers and saccharides to minimize the cyclization, hydrolysis, and coloration of ACE inhibitors. In the examples of the Harris et. al. patent, quinapril hydrochloride is used as the ACE inhibitor, which is stabilized using exclusively magnesium carbonate as the stabilizer in combination with lactose, which is used as the saccharide. Like the Sherman et. al. patents discussed previously, Harris et. al. are using a dry-mix and subsequent wet granulation process. Harris et. al. purport that their process results in storage-stable quinapril hydrochloride compositions based on relatively mild testing conditions. Stability at adverse temperature and humidity conditions of storage is not discussed by Harris.

Furthermore, patents claiming storage-stable ACE inhibitor compositions do not ensure that the same compositions would be simultaneously "bio-stable," e.g., by not extensively hydrolyzing in aqueous environments.

As such, there remains a long-standing need for manufacturable and simultaneously storage-stable and bio-stable formulations and methods of preparation of ACE inhibitors. There is a further need for formulations and methods of manufacturing of products of ACE inhibitors that minimize breakdown of the product, that are inexpensive and time-efficient, and that have improved uniformity from batch to batch. Additionally, there is a need for methods of preparation and formulations of ACE inhibitors which are greatly reduced in breakdown products during preparation, subsequent storage, and/or subsequent dissolution in water.

### SUMMARY OF THE INVENTION

The present invention relates to manufacturable and simultaneously storage-stable and bio-stable formulations of ACE inhibitors, especially enalapril maleate and similar salts, quinapril hydrochloride and similar salts, and similar drugs which can undergo degradation due to hydrolysis and cyclization. The present invention also relates to time-efficient methods of preparing storage-stable and bio-stable formulations thereof. Further, the present invention provides formulations and methods of preparation of ACE inhibitors that minimize breakdown of the product preparation ("manufacturable products"), subsequent storage ("storage-stable products"), and/or subsequent dissolution in water ("bio-stable products") thereof. The present invention also relates to products of the methods of preparing storage-stable and bio-stable formulations of ACE inhibitors. The present invention also provides storage-stable and bio-stable formulations of ACE inhibitors substantially free of harmful and/or undesired breakdown products. It is now possible to prepare such formulations of ACE inhibitors, which are substantially free of these contaminants.

Methods of treatment of cardiovascular disorders, such as hypertension, are also provided by the present invention wherein stabilized formulations of an ACE inhibitor, for example enalapril maleate or quinapril hydrochloride, are administered to patients.

These and other embodiments of the present invention will readily occur to those of ordinary skill in the art in view of the disclosure herein.

The ACE inhibitors of the present invention are selected from the group consisting of enalapril maleate, quinapril hydrochloride, benazepril hydrochloride, moexipril hydrochloride, lisonopril hydrochloride, ramipril hydrochloride, indopril hydrochloride, and pharmaceutically acceptable salts thereof.

### BRIEF DESCRIPTION OF THE DRAWINGS

Figure 1 depicts stability profiles of different formulations of stabilized enalapril maleate.

Figure 2 depicts impurity levels in different formulations of stabilized enalapril maleate.

Figure 3 depicts stability profiles of different tablet formulations of stabilized quinapril hydrochloride.

Figure 4 depicts impurity levels in different formulations of stabilized quinapril hydrochloride.

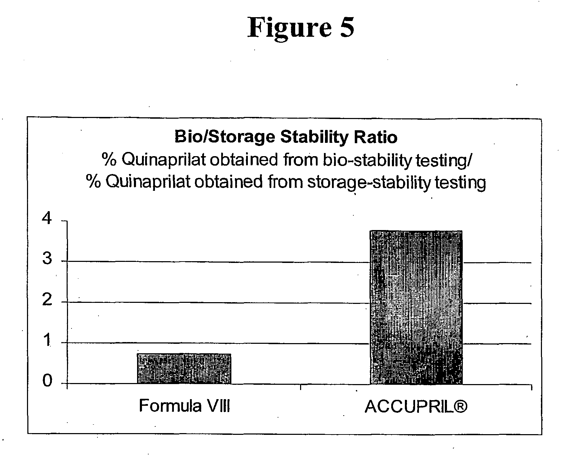

Figure 5 depicts the Bio/Storage Stability Ratios (BSSR-values) of Formula VIII of the present invention and commercial formulation ACCUPRIL®.

### DETAILED DESCRIPTION OF THE INVENTION

The practice of the present invention employs, unless otherwise indicated, conventional methods of chemistry and drug synthesis and formulation, all within the skill of the art. Such techniques are explained fully in the literature. See, e.g., Remington's Pharmaceutical Sciences, 18th Edition (Easton, Pennsylvania: Mack Publishing Company, 1990), incorporated herein by reference.

The present invention arises from the surprising discovery that it is possible to provide for the rapid, economical preparation of ACE inhibitors while minimizing breakdown of the product during manufacturing, storage and dissolution in water, and maximizing the final purity of that product. Breakdown may be due to factors including, but not limited to, hydrolysis and cyclization. Cyclization may be due to factors including, but not limited to, internal nucleophilic attack. Storage-stable and bio-stable formulations of stabilized ACE inhibitors, especially those of enalapril maleate and quinapril hydrochloride, are also provided. Although not wishing to be bound by theory, the inventor believes that the presence of alcohol alone or in combination with alkali and/or alkali-earth metals, as described hereinafter, not only accelerates the manufacture of the product but also minimizes extensive hydrolysis and/or cyclization of the product during production, storage, and dissolution in aqueous environments. Surprisingly, it has also been found that the presence of cellulosic materials in the present method and the non-inclusion of any saccharides results in manufacturable and simultaneously storage-stable and bio-stable formulations that are substantially free of breakdown products; in the case of enalapril maleate, resulting in formulations which are substantially free of enalaprilat and/or enalapril-DKP (the hydrolysis and cyclization products of enalapril maleate, respectively), or, in the case of quinapril hydrochloride, substantially free of quinaprilat and/or quinapril-DKP (the hydrolysis and cyclization products of quinapril hydrochloride, respectively).

Furthermore, the present invention arises from the surprising discovery that the manufacturability and stability of formulations of ACE inhibitors are markedly enhanced by minimizing the contact time of the ACE inhibitor with water during the manufacturing process as described hereinafter. Methods according to the present invention ensure minimal contact time between ACE inhibitors and water, while at the same time achieving intimate intermixing of the molecules of the ACE inhibitor and the metal compound. For example, in some embodiments, the ACE inhibitor, for example enalapril maleate, is first dissolved or dispersed in ethanol and then is contacted with the metal compound molecules via the addition of an aqueous solution of the metal compound to the alcoholic dispersion of the ACE inhibitor. Such methodology essentially eliminates the contact of enalapril maleate with water alone since ethanol is always present during the preparation, thereby not only accelerating the suspension or dispersion of the ACE inhibitor but also minimizing the deleterious hydrolytic effects of water on the stability of enalapril maleate. However, in the case of ACE inhibitors that can not be easily dispersed in ethanol such as quinapril hydrochloride, it is more preferred to first dissolve or disperse the ACE inhibitor in a solution of ethanol and water, namely, a hydroalcoholic solution, and then add the metal compound to such hydroalcoholic solution or dispersion of the ACE inhibitor to achieve intimate intermixing of the ACE inhibitor and the metal compound molecules.

In a preferred method of preparation, a metal compound can first be dissolved or dispersed into a hydroalcoholic mixture, and then an ACE inhibitor can be easily dispersed into the hydroalcoholic solution or dispersion of the metal compound. As described in examples of quinapril hydrochloride formulations hereinafter, such a method drastically minimizes the time to produce a clear solution and intimate mixing between the molecules of the ACE inhibitor and the metal compound, while at the same time significantly reducing the contact time of the ACE inhibitor with the water of the hydroalcoholic mixture.

Finally, the present invention arises from the surprising discovery that the compositions and methodologies described herein result in formulations of ACE inhibitors that are not only acceptably manufacturable on an industrial scale but also, they are desirably stable during storage at room temperature and other adverse conditions ("storage-stable products"), while at the same time and as compared to current commercially available products, such formulations of stabilized ACE inhibitors do not hydrolyze extensively when placed in water for a certain amount of time ("bio-stable products"). Specifically, it has been now discovered that, in the case of the ACE inhibitor quinapril hydrochloride which can not be quickly dispersed in ethanol alone, the preferred methodology of first mixing the metal compound with a mixture of water and ethanol (hydroalcoholic system) and then adding the quinapril hydrochloride to very quickly produce a clear solution, results in stabilized quinapril hydrochloride formulations that do not only possess superior storage stability characteristics but also, they simultaneously demonstrate significantly improved bio-stability properties as compared to the currently commercial brand product ACCUPRIL® Tablets (Parke-Davis).

As presented in the examples below, when quinapril hydrochloride tablets prepared according to the present invention were placed for three hours in purified water maintained at 37°C, not more than about 0.7% by weight of the quinapril hydrochloride was converted to its hydrolysis byproduct quinaprilat. On the other hand when the same experiment was repeated with the brand ACCUPRIL® Tablets, about 3.4% by weight of quinapril hydrochloride converted to quinaprilat. Furthermore, when quinapril hydrochloride tablets prepared according to the present invention were first stored for 10 days at 60°C and 75% relative humidity and then placed for three hours in water maintained at 37°C, not more than about 4% by weight of quinapril hydrochloride was hydrolyzed to quinaprilat. On the other hand, when the same experiment was repeated with ACCUPRIL® Tablets that were similarly storage-stressed, more than about 11% by weight of quinapril hydrochloride converted to quinaprilat.

Hydrolysis products of ACE inhibitors, for example enalaprilat and quinaprilat, are poorly absorbed from the gastrointestinal tract. As such, it is desirable to provide formulations of stabilized ACE inhibitors, for example the stabilized quinapril hydrochloride presented herein, which are simultaneously storage-stable and bio-stable because such stabilized formulations are more likely to achieve desired bioavailability levels and therapeutic objectives of this drug category than formulations that are no so stabilized.

As used herein, the phrase "stabilized ACE inhibitor" refers to storage-stable and bio-stable ACE inhibitors prepared according to the present invention, and is meant to encompass an ACE inhibitor salt with a metal compound or an ACE inhibitor in close proximity to the molecules or metal cations of alkali or alkali-earth metal salts. As used herein, the term "DKP" or "diketopiperazine" includes DKP-compounds of the ACE inhibitor, which are the cyclization degradation by-products of the ACE inhibitor. For example, the DKP Breakdown products of enalapril maleate and quinapril hydrochloride, enalapril-DKP and quinapril-DKP, respectively, are encompassed by the term "DKP" or "diketopiperazine".

The term "substantially free" refers to compositions that have significantly reduced levels of detectable breakdown products, e.g. enalaprilat and/or enalapril-DKP in the case of enalapril maleate, or quinaprilat and/or quinapril-DKP in the case of quinapril hydrochloride. In one embodiment, the stabilized enalapril maleate contains less than about 5% by weight enalaprilat, preferably less than 2.5% by weight enalaprilat, or, even more preferably, less than about 1% by weight, or, in the case of other stabilized ACE inhibitors, a similarly small quantity of analogous impurity. In another embodiment, the stabilized enalapril maleate contains less than about 1.0% by weight DKP, more preferably less than about 0.5% by weight DKP, or, even more preferably, less than about 0.25% by weight DKP or, in the case of other stabilized ACE inhibitors, a similarly small quantity of analogous impurity.

In another embodiment, the stabilized quinapril hydrochloride contains less than about 7.5% by weight quinaprilat, preferably less than about 5% by weight quinaprilat, more preferably less than about 2.5% by weight quinaprilat, or, even more preferably, less than about 1% by weight quinaprilat, or, in the case of other stabilized ACE inhibitors, a similarly small quantity of analogous impurity. In another embodiment, the stabilized quinapril hydrochloride contains less than about 5.0% by weight DKP, preferably less than about 1% by weight DKP, more preferably less than about 0.5% by weight DKP, or, even more preferably, less than about 0.25% by weight DKP, or, in the case of other stabilized ACE inhibitors, a similarly small quantity of analogous impurity. In another embodiment, the stabilized quinapril hydrochloride contains less than about 5% by weight quinaprilat and less than about 1% by weight DKP, or, in the case of other stabilized ACE inhibitors, similarly small quantities of analogous impurities.

In yet another embodiment, when placed for three hours in purified water maintained at 37°C, the freshly prepared stabilized quinapril hydrochloride hydrolyzes into less than about 3.5% by weight quinaprilat, more preferably less than about 2.5% by. weight quinaprilat, or even more preferably than about 1.5% by weight quinaprilat, or, in the case of other stabilized ACE inhibitors, a similarly small quantity of analogous impurity.

In another embodiment, after being stored for 10 days at 60°C and 75% RH and when placed for three hours in purified water maintained at 37°C, the stabilized quinapril hydrochloride hydrolyzes into less than about 11% by weight quinaprilat, more preferably less than about 8% by weight quinaprilat, or even more preferably less than about 5% by weight quinaprilat, or, in the case of other stabilized ACE inhibitors, a similarly small quantity of analogous impurity.

The present invention also provides compositions of ACE inhibitors which can be simultaneously storage-stable and bio-stable. A Bio/Storage Stability Ratio (BSSR-value) of the stabilized ACE inhibitors can be calculated by using, in the numerator, the levels of the hydrolytic degradation product of the ACE inhibitor obtained from a bio-stability assessment divided by, in the denominator, the levels of hydrolytic degradation product of the ACE inhibitor obtained from a storage-stability assessment. In assessing bio-stability, a pharmaceutical tablet, which can be either freshly prepared or previously subjected to storage-stability aging, can be dissolved or contacted with water maintained at 37°C for 3 hours and then levels of hydrolytic degradation products can be analyzed. Storage-stability assessment can be taken from a 3-month timepoint of an accelerated storage-stability study conducted at 40°C and 75% relative humidity (RH) and then levels of hydrolytic degradation products can be analyzed. Preferably, simultaneously storage-stable and bio-stable compositions of ACE inhibitors can be those formulations of ACE inhibitors which do not contain more than 5% of the hydrolytic breakdown product of the ACE inhibitor while at the same time displaying a Bio/Storage Stability Ratio lower than about 3.5 (which is lower than the BSSR-value of 3.78 obtained by the commercial ACCUPRIL® brand product).

As used herein, the terms "analogous breakdown product", "degradation product", or "analogous impurity" or derivatives thereof, refer to undesired contaminants formed by breakdown of an ACE inhibitor which are similar, as appreciated by persons of ordinary skill in the art, to those resulting from ACE inhibitor breakdown. Breakdown of ACE inhibitors may be caused by factors including, but not limited to, hydrolysis and cyclization.

The present invention provides methods of preparing storage-stable and bio-stable formulations of ACE inhibitors, especially enalapril maleate and quinapril hydrochloride. The methods comprise the steps of mixing an ACE inhibitor, for example enalapril maleate, with an alcohol to form an alcoholic dispersion of the ACE inhibitor, dissolving or dispersing a metal compound in water to form a metal compound solution or dispersion, and mixing together the alcoholic dispersion, of the ACE inhibitor and the aqueous solution or dispersion of the metal compound to form a "final hydroalcoholic solution or dispersion" of the ACE inhibitor and the metal compound. In another embodiment, the methods comprise the steps of mixing an ACE inhibitor, for example quinapril hydrochloride, with a mixture of alcohol and water (hydroalcoholic system) to form a hydroalcoholic dispersion of the ACE inhibitor and adding therein a metal compound under constant mixing to form a "final hydroalcoholic solution or dispersion" of the ACE inhibitor and the metal compound.

In other preferred embodiments, especially in the case of stabilized quinapril hydrochloride formulations, the methods comprise the steps of dissolving or dispersing the metal compound into a mixture of alcohol and water (hydroalcoholic system) to form a hydroalcoholic solution or dispersion of the metal compound, and then adding slowly therein an ACE inhibitor, such as quinapril hydrochloride powder, under constant mixing to form a "final hydroalcoholic solution or dispersion" of the ACE inhibitor and the metal compound.

In some embodiments, the various "final hydroalcoholic solutions or dispersions" of the ACE inhibitor and the metal compound produced from the methods discussed previously are mixed continuously until they become converted to clear solutions free of any foamation. In other embodiments the methods further comprise adding at least one excipient to the clear solutions. Alternative embodiments further comprise adding one or more antioxidants to the alcoholic or hydroalcoholic dispersions. Some embodiments further comprise blending at least one excipient and the clear solution to form a granulate. In other embodiments, the granulates are dried and preferably processed into a pharmaceutical solid, e.g. tablet, capsule, particulate and the like.

As used herein, the term "alcohol" refers to lower, e.g. C1 to C6, monohydric alcohols acceptable for pharmaceutical preparations, especially ethanol. While polyhydric alcohols may be used, they are generally more toxic and are not preferred. The terms "alcohol" and "alcoholic" include water/alcohol mixtures — hydroalcoholic systems.

As used herein, the term "metal compound" refers to a compound added to the ACE inhibitor to effect its conversion to the stabilized ACE inhibitor. Metal compounds useful in connection with this invention are basic salts of alkali and alkaline earth metals which are readily water soluble and which do not interfere with the stability of the compositions of the present invention. Thus, the readily water and/or alcohol soluble salts of lithium, sodium, potassium, cesium, rubidium, calcium, magnesium, strontium and barium, bicarbonate, carbonate, hydroxide, oxide, acetate, borate and similar materials may be employed herein as the metal compound. Preferred among these are sodium, potassium, calcium, and magnesium salts, especially sodium salts. Counterions which are preferred are bicarbonate, hydroxide and carbonate, with bicarbonate being most preferred. Sodium bicarbonate is most preferred for certain embodiments of the invention. This includes, but is not limited to, sodium bicarbonate, sodium hydroxide, sodium acetate, and sodium borate. While sodium is the conventional and preferred ion, potassium and other pharmaceutically acceptable cations may be employed and all such will be understood to be encompassed hereby.

As used herein, the term "antioxidant" refers to a composition which reduces or prevents oxidation. "Antioxidants" include, but are not limited to, butyl hydroxyl anisol (BHA), butyl hydroxyl toluene (BHT), maleic acid, and ascorbic acid. In a preferred embodiment the antioxidant is maleic acid, and is present in an amount from about 0.001% to about 2.0% w/w per unit dose.

In one embodiment of the present invention, the method further comprises the addition and/or dissolution of a thickening agent to the "final hydroalcoholic solution or dispersion" or to the clear solution of the ACE inhibitor and the metal compound. As used herein, the term "thickening agent" is well known to those of skill in the art. A wide variety of thickening agents may be used to prepare the stable formulations of the present invention. Suitable thickening agents include any and all biocompatible polymers known to function as thickening agents, binding aids, coprecipitation vehicles/aids, or solid solution/dispersion agents. In a preferred embodiment of the present invention, the thickening agent is selected from the group consisting of solid or liquid polyethylene glycols, propylene glycol, glycerin, cross-linked povidone, polyvinylpyrrolidone, polyvinyl alcohol, and modified celluloses known to form hydrocolloids, such as hydroxypropylmethylcellulose, methylcelullose, hydroxypropylcellulose, and others. In a more preferred embodiment, the thickening agent is polyvinyl pyrrolidone (PVP), and is present in an amount from about 1% to about 10% w/w per unit dose. With being bound by theory, the inventor believes that the thickening agent serves to maintain the molecules of the ACE inhibitor intimately intermixed with the molecules or metal cations of the metal compound.

Tabletting and other pharmaceutically acceptable excipients may be blended with the dry material provided hereby to facilitate formation of conventional and convenient pharmaceutical solids. Such formulation is known *per se.*

As used herein, the term "clear solution" refers to the solution or the final hydroalcoholic solution or dispersion of the ACE inhibitor and the metal compound attained after complete or substantially complete conversion of the ACE inhibitor to the stabilized ACE inhibitor wherein the molecules of the ACE inhibitor are intimately mixed with the molecules or the metal cations of the metal compound. The term "clear solution" may refer to a substantially clear material having some coloration, typically a yellowish tint, which may appear to be colloidal. This definition includes solutions which are partly cloudy. For example, as used for the end-point for complete conversion of enalapril maleate to stabilized enalapril maleate or for the complete conversion of quinapril hydrochloride to stabilized quinapril hydrochloride, the term "clear solution" refers to the relative absence of foamation or bubbling. The presence of a "clear solution" is measured by eye, assessing the absence of foamation.

The term "excipient" includes, but is not limited to, the family of modified celluloses such as carboxymethyl and ethyl cellulose, hydroxyethyl cellulose, hydroxypropyl cellulose, microcrystalline cellulose, cross-linked povidone, hydroxypropylmethylcellulose and others. In one embodiment, the excipient is at least one of microcrystalline cellulose, starch, and sodium starch glycolate.

In one embodiment of the present invention, a pharmaceutical preparation comprising a pharmaceutically acceptable salt of a stabilized ACE inhibitor substantially free of breakdown products is provided. In a preferred embodiment, the ACE inhibitor is enalapril maleate, the stabilized ACE inhibitor comprises enalapril maleate molecules in close proximity to sodium cations, and the breakdown products are enalaprilat and/or enalapril-DKP. In another preferred embodiment, the ACE inhibitor is quinapril hydrochloride, the stabilized ACE inhibitor comprises quinapril hydrochloride molecules in close proximity to sodium cations, and the breakdown products are quinaprilat and/or quinapril-DKP.

In still another embodiment of the present invention, pharmaceutical preparations are provided comprising a pharmaceutically acceptable salt of a stabilized ACE inhibitor and microcrystalline cellulose, substantially free of breakdown products. In a preferred embodiment, the ACE inhibitor is enalapril maleate or quinapril hydrochloride, the stabilized ACE inhibitor comprises enalapril maleate or quinapril hydrochloride molecules in close proximity to sodium cations, and the breakdown products are enalaprilat and/or enalapril-DKP, or quinaprilat and/or quinapril-DKP.

Microcrystalline cellulose is known *per se* and a variety of such are commercially available. Exemplary among these is the family of products sold by the FMC Corporation under the trademark Avicel®. Any of the members of this family may be used in connection with the practice of one or more embodiments of the present invention and all are contemplated hereby. Other cellulose products which are similar in nature to microcrystalline cellulose may find utility herein, such as parenchymal cell cellulose, powdered cellulose, and the like.

In addition to the preferred microcrystalline celluloses and similar materials, other cellulosic materials may also be employed in connection with one or more embodiments of the present invention. Thus, modified celluloses such as methyl cellulose, hydroxyethyl cellulose, hydroxypropyl cellulose, methylhydroxyethyl cellulose, methylhydroxypropyl cellulose, carboxymethyl cellulose salts and esters, (e.g. sodium, potassium etc. salts), and other cellulose derivatives may be so employed. It will be appreciated by persons of ordinary skill in the art that such cellulosic materials should be consistent with the overall spirit of the invention. Thus, such materials may be employed which do not adversely effect the processing set forth herein and which do not interfere with the stability of the resulting products.

Those of skill in the art will also understand that the term "excipient" is used colloquially to include such agents as disintegrating agents, carriers, diluents, pigments, binders, colorants, and lubricants. In one embodiment, the excipient is a disintegrating agent.

The term "disintegrating agent" is well known to those of skill in the art as an agent that enhances the conversion of a compact material into fine primary particles during dissolution. Disintegrating agents include, but are not limited to, starch, cellulose, sodium starch glycolate, cross-linked povidones, and modified celluloses, and are present in amounts from about 1% to about 25% w/w per unit dose.

The term "lubricant" is well known to those of skill in the art as an additive to prevent the sticking of the formulation to tooling during the tabletting process. Lubricants include, but are not limited to, stearates, hydrogenated vegetable oils, and talc. In some embodiments of the present invention, the lubricant is a stearate. In some preferred embodiments, the lubricant is magnesium stearate or glyceryl monostearate and is present in an amount from about 0.5% to about 10% w/w per unit dose. In a more preferred embodiment, the lubricant is magnesium stearate and is present in an amount from about 0.01% to about 1% w/w per unit dose.

The term "binder" is well known to those of skill in the art as an agent that holds the components of the formulation together. Binders include, but are not limited to, gelatin, polyvinylpyrrolidone (PVP), hydroxypropylmethylcellulose (HPMC), starch grades (pregelatinized or plain), hydroxypropylcellulose (HPC), and carboxymethylcellulose (CMC), and their salts.

As used herein, the term "drying" refers to the substantial removal of liquid from the granulation. Drying may be accomplished in a number of manners well known to those of skill in the art including, but not limited to the use of ovens, fluid bed driers, and other similar equipment. In a preferred embodiment, the granulation is dried for about 12 hours at 50°C to substantially remove liquid from the granulation.

As used herein, the term "pharmaceutical solid dosage forms" refers to the final solid pharmaceutical product. The tenn "pharmaceutical solid dosage form" includes, but is not limited to, tablets, caplets, beads, and capsules (including both hard shell capsules and soft gelatin capsules).

The processes of mixing, drying, granulating and making pharmaceutical solid formulations are well known to those of skill in the art. See, e.g., Theory & Practice of Industrial Pharmacy, 3rd Edition, Liberman, Lachman, and Kanig, eds. (Philadelphia, Pennsylvania: Lea & Febiger), incorporated herein by reference.

By "pharmaceutically acceptable" or "pharmacologically acceptable" is meant a material which is not biologically or otherwise undesirable, i.e., the material can be administered to an individual along with the stabilized ACE inhibitor formulation without causing any undesirable biological effects or interacting in a deleterious manner with any of the components of the formulation in which it is contained.

### EXAMPLES

Below are several examples of specific embodiments for carrying out the present invention. The examples are offered for illustrative purposes only.

### Example 1

In the following formulations, the quantities of ingredients are provided in equivalent weights (in mg per unit dose (mg/ud)). The approximate batch size was about 6000 units.

### Preparation of Formula I

Enalapril maleate (20 mg/ud; Byron Chem. Co., Long Island City, NY) was suspended in SD3A denatured alcohol (50 mg/ud) with stirring at 500 rpm. Full dispersion of the enalapril maleate in the alcohol was achieved in less than about 10 seconds. In a separate container, sodium bicarbonate (11 mg/ud) and povidone (polyvinylpyrrolidone; Plasdone®, ISP, Bound Brook, New Jersey) were dissolved in 100 mg/ud purified water (USP). The sodium bicarbonate/povidone solution was added gradually to the alcoholic drug dispersion with constant stirring (200 rpm) until a clear solution was achieved to yield solution 1, e.g. the solution was free of foaming (bubbling).

Microcrystalline cellulose (225 mg/ud, Avicel® PH200; FMC Corporation, Philadelphia, PA), sodium starch glycolate (30 mg/ud, Explotab®; Edward Mendell Co., New York, NY), and silicon dioxide (8 mg/ud; Syloid ® 244 FP; W.R. Grace & Co., Baltimore, MD) were mixed for three minutes in a high shear mixer for 3 minutes (Collete Gral 10, Machines Collette, Belgium) to yield mixture 1. Mixture 1 was blended with solution 1 for three minutes at low speed with the choppers set to low. The resulting granulation was then dried for 12 hours at 50°C. The dried granulation was then passed through a #30 mesh screen and blended with magnesium stearate (2 mg/ud), producing the final tabletting blend of Formula I.

### Preparation of Formula II

Formula II was synthesized according to the methods set forth for Formula I with the following variation. Enalapril maleate was suspended in SD3A denatured alcohol (30 mg/ud) and Tween80® (polysorbate 80; 20 mg/ud; Sigma, St. Louis, MO) with stirring at 500 rpm.

### Preparation of Formula III

Enalapril maleate (20 mg/ud; Byron Chem. Co., Long Island City, NY) was suspended in purified water (50 mg/ud, USP) with stirring at 500 rpm. In a separate container, sodium bicarbonate (11 mg/ud) and povidone (9 mg/ud; PVP K29/32) were dissolved in purified water (100 mg/ud, USP). The sodium bicarbonate/povidone solution was added gradually to the drug dispersion with constant stirring (200 rpm) until an almost clear solution was achieved to yield solution 2.

Microcrystalline cellulose (225 mg/ud; Avicel® PH200; FMC, Philadelphia, PA), sodium starch glycolate (30 mg/ud, Explotab®; Edward Mendell Co., New York, NY), and silicon dioxide (8 mg/ud, Syloid® 244 FP; W.R. Grace & Co., Baltimore, MD) were mixed for three minutes in a high shear mixer for 3 minutes (Collete Gral 10) to yield mixture 2. Mixture 2 was blended with solution 2 for three minutes at low speed with the choppers set to low. The resulting granulation was then dried for 12 hours at 50°C. The dried granulation was then passed through a #30 mesh screen and blended with magnesium stearate (2 mg/ud), producing the final tabletting blend of Formula III.

### Preparation of Formula IV

Formula IV was synthesized according to the methods set forth for Formula III with the following variation. Enalapril maleate was suspended in purified water (30 mg/ud) and Tween® 80 (20 mg/ud; Sigma, St. Louis, MO) with stirring at 500 rpm.

Formulations I and II were made according to the present invention, while Formulations III and IV were made according to Merslavic et al. in terms of conversion of enalapril maleate to enalapril sodium. However, unlike the methods described in Merslavic et al., Formulations III and IV were prepared using microcrystalline cellulose instead of starch and cellulose as the diluent. Formulation IV further contained Tween 80® dispersed in water to increase the wetting properties of enalapril maleate.

### Preparation of Formula V

Purified water (50 mg/ud, USP) was mixed with SD3A denatured alcohol (40 mg/ud) in a glass beaker using a Lightnin Mixer at 500 rpm. Quinapril HCl (20 grams, Gyma Laboratories, Westbury, NY) was mixed into the water/alcohol mixture using a Lightnin Mixer at 500 rpm to yield suspension 1. Sodium bicarbonate (11 mg/ud) was slowly added to suspension 1 with stirring using a Lightnin Mixer at 500 rpm until a clear to almost clear solution was attained, with no bubbling or foaming. Povidone (9 mg/ud, PVP K29/32) was then added to the clear/almost clear solution and mixed using a Lightnin Mixer until a uniform solution was attained. Syloid® (30 mg/ud, 244 FP) was then added and mixed until a uniform solution was attained to form solution 1.

Microcrystalline cellulose (70 mg/ud, Avicel® PH101; FMC Corporation, Philadelphia, PA) was mixed with sodium starch glycolate (15 mg/ud, Explotab®; Edward Mendell Co., New York, NY) were mixed for five minutes in a high shear mixer (Collete Gral 10) to form mixture 1. The entirety of solution 1 was then added at once to mixture 1 and granulated for 5 minutes in a high shear mixer (Collete Gral 10), with the paddles and choppers set to low speed.

The resulting granulation was then dried for 10 hours at 45°C-50°C. The dried granulation was then passed through a 0.065 RD stainless steel screen using a Fitzmill Comminutor (Model L1A; Fitzpatrick Co., Elmhurst, IL) set at high speed, producing blend 1, also named Formula V powder form.

Microcrystalline cellulose (95 mg/ud) and sodium starch glycolate (3 mg/ud) were added to blend 1 in a 16 quart Gemco blender (double cone mixer; Gemco) and mixed for 5 minutes to yield blend 2. Magnesium stearate (2 mg/ud; NF; Mallinckrodt Inc., St Louis, MO) was passed through a #30 mesh stainless steel screen and then added to blend 2 and mixed for 1 minute to yield blend 3. Blend 3 was compressed into tablets of about 250 mg weight, also named Formula V tablet form. The tablets had an approximate hardness of 15-20kp, measured using a Schleuinger Model D6 hardness tester (Dr. Schleuinger Pharmatrone, Manchester, NH).

### Preparation of Formula VI

Purified water (75 mg/ud, USP) was mixed with SD3A denatured alcohol (40 mg/ud) in a glass beaker using a Lightnin Mixer at 500 rpm. Quinapril HCl (20 mg/ud) were mixed into the water/alcohol mixture using a Lightnin Mixer at 500 rpm to yield suspension 1. Sodium bicarbonate (11 mg/ud) was slowly added to suspension 1 with stirring using a Lightnin Mixer at 500 rpm until a clear to almost clear solution was attained, with no bubbling or foaming. Povidone (9 mg/ud, PVP K29/32) was then added to the clear/almost clear solution and mixed using a Lightnin Mixer until a uniform solution was attained, solution 1.

Microcrystalline cellulose (100 mg/ud, Avicel® PH101; FMC Corporation, Philadelphia, PA) was mixed with sodium starch glycolate (3 mg/ud, Explotab®; Edward Mendell Co., New York, NY) were mixed for five minutes in a high shear mixer (Collete Gral 10) to form mixture 1. The entirety of solution 1 was then added at once to mixture 1 and granulated for 5 minutes in a high shear mixer (Collete Gral 10), with the paddles and choppers set to low speed.

The resulting granulation was then dried for 10 hours at 45°C-50°C. The dried granulation was then passed through a 0.065 RD stainless steel screen using a Fitzmill Comminutor (Model L1A) set at high speed, producing blend 1, also named Formula VI powder form.

Microcrystalline cellulose (95 mg/ud) and sodium starch glycolate (3 mg/ud) were added to blend 1 in a 16 quart Gemco blender (double cone mixer; Gemco) and mixed for 5 minutes to yield blend 2. Magnesium stearate (2 mg/ud; NF) was passed through a #30 mesh stainless steel screen and then added to blend 2 and mixed for 1 minute to yield blend 3. Blend 3 was compressed into tablets of about 250 mg weight with an approximate hardness of 15-20kp, also named Formula VI tablet form.

### Preparation of Formula VII

Purified water (15.5 mg/ud, USP) was mixed with SD3A denatured alcohol (40 mg/ud) in a glass beaker using a Lightnin Mixer at 500 rpm. Quinapril HCl (20 mg/ud) were mixed into the water/alcohol mixture using a Lightnin Mixer at 500 rpm to yield suspension 1. Sodium bicarbonate (11 mg/ud) was slowly added to suspension 1 with stirring using a Lightnin Mixer at 500 rpm until a clear to almost clear solution was attained, with no bubbling or foaming. Povidone (9 mg/ud, PVP K29/32) was then added to the clear/almost clear solution and mixed using a Lightnin Mixer until a uniform solution was attained, solution 1.

Microcrystalline cellulose (95 mg/ud, Avicel® PH101; FMC Corporation, Philadelphia, PA) was mixed with sodium starch glycolate (15 mg/ud, Explotab®; Edward Mendell Co., New York, NY) were mixed for five minutes in a high shear mixer (Collete Gral 10) to form mixture 1. The entirety of solution 1 was then added at once to mixture 1 and granulated for 5 minutes in a high shear mixer (Collete Gral 10), with the paddles and choppers set to low speed.

The resulting granulation was then dried for 10 hours at 45°C-50°C. The dried granulation was then passed through a 0.065 RD stainless steel screen using a Fitzmill Comminutor (Model L1A) set at high speed, producing blend 1, also named Formula VII powder form.

Microcrystalline cellulose (95 mg/ud) and sodium starch glycolate (3 mg/ud) were added to blend 1 in a 16 quart Gemco blender and mixed for 5 minutes to yield blend 2. Magnesium stearate (2 mg/ud; NF) was passed through a #30 mesh stainless steel screen and then added to blend 2 and mixed for 1 minute to yield blend 3. Blend 3 was compressed into tablets of about 250 mg weight with an approximate hardness of 15-20kp, also named Formula VII tablet form.

### Preparation of Formula VIII

Formula VIII along with its derivative Formulations A, B, C and D presented below were manufactured in industrial scale quantities using industrial scale equipment and targeting the production of more than 100,000 tablets of each of the four quinapril hydrochloride strengths. Formula VIII produced first a quinapril hydrochloride intermediate which was then separated in aliquots that were mixed with other excipients to produce Formulations A, B, C and D of different tablet-strengths of quinapril base, namely, 5, 10, 20 and 40 mg, respectively.

Quinapril hydrochloride intermediate was prepared by first mixing 86.112 kg of microcrystalline cellulose and 10.452 kg of sodium starch glycolate in a Collette Gral High Shear Mixer/Granulator for 5 minutes at low speed with the choppers set at low speed.

Then, the quinapril HCl intermediate was prepared by combining 37.622 kg of purified water and 35.3 liters of denatured alcohol in a separate stainless steel container equipped with an air-operated mixer. While stirring the alcohol and water, 6.682 kg of sodium bicarbonate was added to the stainless steel container. After mixing for 15 minutes, 18.096 kg of quinapril hydrochloride was added to the container gradually so that the contents of the container would not bubble over when adding the quinapril hydrochloride. The bag that contained quinapril hydrochloride was cleaned by adding and rinsing the bag with 1.677 kg of sodium bicarbonate. The contents of the bag were added to the stainless steel container. The contents of the stainless steel container was mixed for at least two and a half hours. After two and a half hours and while mixing, 6.981 kg of Povidone namely, polyvinylpyrrolidone (PVP), was added to the container until a clear solution was obtained.

The clear solution of the stainless steel container was combined with the contents of the Colette Gral mixer/granulator. The stainless steel container was rinsed with 6.5 liters of denatured alcohol and the contents of the rinse were then also added to the granulator. The contents of the granulator were then mixed for two and a half minutes with paddles and choppers set on low speed. The bowl was then manually mixed by scraping the top, sides and bottom of the bowl and the blades of the mixer. The contents of the granulator were then mixed for an additional two and a half minutes with paddles and choppers set on low speed.

The contents of the granulator were dried by spreading it evenly on 84 paper lined trays kept at 45 to 55 degrees Centigrade until the calculated average loss on drying was not more than 2.5% and no individual sample exceeded 3.0%. Total drying time was approximately fourteen and a half hours.

The dried granules were milled through a Fitz Mill using knives forward and a number two stainless steel screen. After being sized, the granules were placed in double polyethylene bags. Approximately 20 grams were tested for its potency by confirming the content of quinapril base per gram of granulation. Further, a one hundred gram sample was randomly withdrawn for tap density, bulk density, and particle size analysis.

Tablets of the drug were prepared by blending the dried granules quinapril hydrochloride intermediate with the excipients as listed below. See formulations A-D. Conventional tabletting procedures were then used to obtain tablets possessing acceptable hardness and friability.

| **Formulation A** | **Component** | **mg per Unit Dose** |
|---|---|---|
| | Quinapril HCl Intermediate | 37.037 |
| | Microcrystalline Cellulose | 19.463 |
| | Sodium Starch Glycolate | 3.125 |
| | Magnesium Stearate | 0.375 |

| **Formulation B** | **Component** | **mg per Unit Dose** |
|---|---|---|
| | Quinapril HCl Intermediate | 74.074 |
| | Microcrystalline Cellulose | 38.926 |
| | Sodium Starch Glycolate | 6.25 |
| | Magnesium Stearate | 0.75 |

| **Formulation C** | **Component** | **mg per Unit Dose** |
|---|---|---|
| | Quinapril HCl Intermediate | 148.148 |
| | Microcrystalline Cellulose | 77.852 |
| | Sodium Starch Glycolate | 12.5 |
| | Magnesium Stearate | 1.5 |

| **Formulation D** | **Component** | **mg per Unit Dose** |
|---|---|---|
| | Quinapril HCl Intennediate | 296.296 |
| | Microcrystalline Cellulose | 155.704 |
| | Sodium Starch Glycolate | 25.00 |
| | Magnesium Stearate | 3.00 |

The method of Formula VIII provides a first step of dissolving the metal compound (sodium bicarbonate) in the hydroalcoholic mixture and then a step of addition the quinapril hydrochloride powder into this mixture to produce the final hydroalcoholic dispersion of the ACE inhibitor and the metal compound. This is in contrast to the previous preparations of other quinapril formulas (e.g., Formulas V-VII) wherein the quinapril hydrochloride powder was first dissolved/dispersed in the prepared hydroalcoholic mixture. The method of Formula VIII proceeded very quickly and effectively compared to Formulas V-VII in attaining the desirable clear solution of the stabilized quinapril hydrochloride. Stabilized formulations of quinapril hydrochloride according to the method of Formula VIII were, in turn, formulated with other excipients to produce the final quinapril hydrochloride intermediate of Formula VIII and its derivative Formulations A, B, C, and D.

The method of Formula VIII represents a preferred mode of the invention for industrial scale manufacturing of stabilized quinapril hydrochloride formulations and other ACE inhibitors which can not be easily and quickly dispersed in alcohols not mixed with water. If an ACE inhibitor, as in the case of quinapril hydrochloride, is better dispersed in a hydroalcoholic mixture, compared to alcohol alone, and such mixture is prepared first in the manufacturing process, the ACE inhibitor may take extensive and variable time to be dispersed in such mixture. Consequently, at industrial scale, the contact time of the ACE inhibitor and water is increased along with the possibility for the ACE inhibitor to degrade at higher rates during manufacturing and storage. This phenomenon was observed during the industrial scale-up of Formulas V, VI, and VII, wherein increased times of mixing to obtain the clear solution of quinapril hydrochloride were required, and quite variable and non-reproducible levels of quinaprilat and quinapril-DKP were demonstrated after manufacturing and storage. However, it has been discovered now that the technique presented above for the preparation of Formula VIII eliminated such problems and produced stabilized quinapril hydrochloride formulations possessing excellent manufacturability at industrial scale and reliable reproducibility of their stability properties immediately after production and during storage at various conditions of temperature and humidity.

### Preparation of Formula IX

Quinapril hydrochloride was formulated into lab-scale Formula IX in a manner and ingredient quantities identical to those of Formula VIII and its derivative Formulation D presented above. In contrast to Formula VIII which was manufactured at industrial scale, Formula IX was prepared at a lab-scale of 100 tablets (each of 40-mg strength relative to content of quinapril-base) using lab-scale equipment. Also, in contrast to Formula VIII which contained polyvinyl pyrrolidone (PVP) as the thickening agent or coprecipitation aid used during the manufacturing of the quinapril hydrochloride intermediate, Formula IX contained solid polyethylene glycol 8,000 (PEG-8,000).

### Preparation of Formula X

Quinapril hydrochloride was formulated into lab-scale Formula X in a manner and ingredient quantities identical to those of Formula VIII and its derivative Formulation D presented above. In contrast to Formula VIII which was manufactured at industrial scale, Formula X was prepared at a lab-scale of 100 tablets (each of 40-mg strength relative to content of quinapril-base) using lab-scale equipment. Also, in contrast to Formula VIII which contained polyvinyl pyrrolidone (PVP) as the thickening agent or coprecipitation aid used during the manufacturing of the quinapril hydrochloride intermediate, Formula X contained polyvinyl alcohol (PVA).

### Example 2

### Comparison of stability profiles of different formulations of enalapril maleate.

The stability profiles of different formulations of enalapril maleate were compared. The stability of formulations of stabilized enalapril maleate (Formulas I-IV, as described above) were also compared to a commercial formulation of enalapril maleate, VASOTEC™ (Merck & Co.) referred to as "Enalapril- commercial." Formulations were stored at 60°C with 75% relative humidity to simulate extended storage. Stability of the formulations was assessed at 5, 10, and 15 days by HPLC.

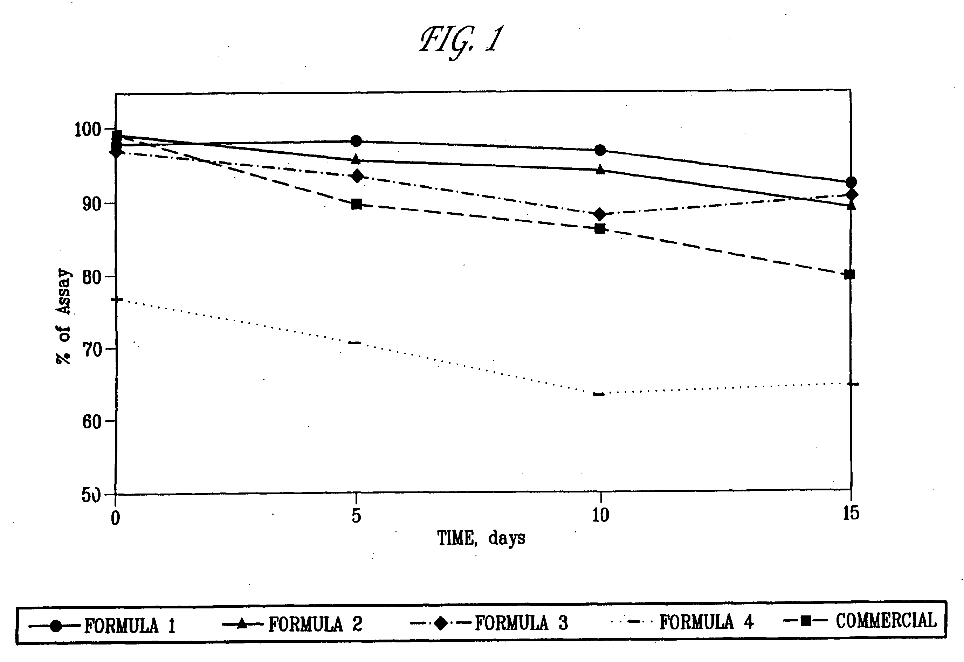

As shown in Figure 1, Formulation I was more stable than the VASOTEC™ formulation and Formulations II-IV at the 5, 10, and 15 day timepoints. At the 5 and 10 day timepoints, Formulation II exhibited greater stability than Formulations III, IV, and the VASOTEC™ formulation, referred to as the "Enalapril- commercial." Formulation II was more stable at the 5, 10, and 15 day timepoints than the VASOTEC™ formulation and Formulation IV.

### Example 3

### Comparison of levels of impurities in different formulations of enalapril maleate.

The levels of impurities in different formulations of enalapril maleate were compared. The level of impurities of the formulations of stabilized enalapril maleate were also compared to a commercial formulation of enalapril maleate, VASOTEC™. Formulations were stored at 60°C with 75% relative humidity to simulate extended storage. Impurity levels of the formulations were assessed at 5, 10, and 15 days by measurement of enalaprilat and enalapril-DKP formation by HPLC.

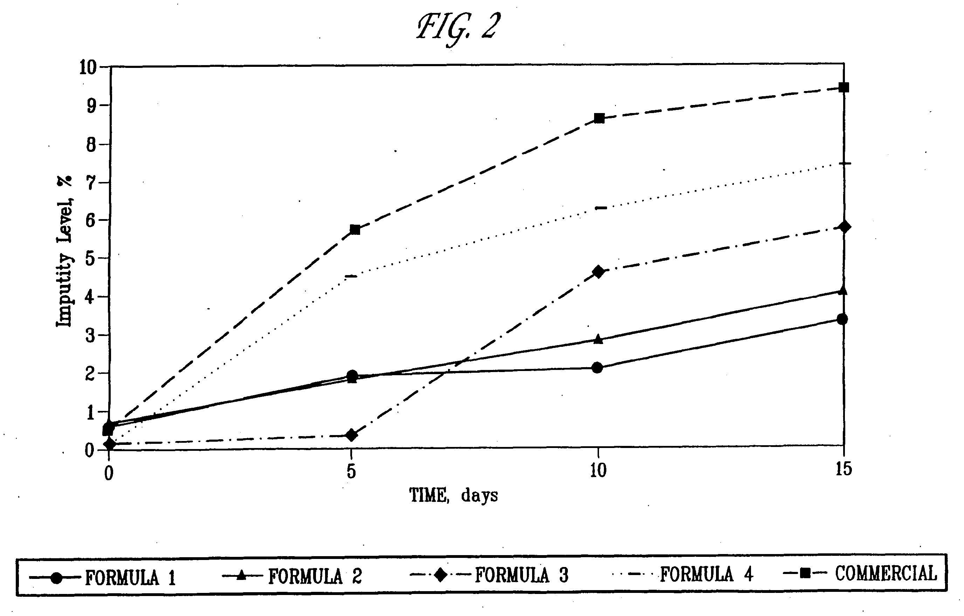

As shown in Figure 2, at the 10 and 15 day timepoints, Formulation I exhibited the greatest purity; e.g. the lowest level of impurity. At the 10 and 15 day timepoints, Formulation I had less impurities than did Formulations III, IV, and VASOTEC™.

Formulation II exhibited less impurities than did Formulations III, IV, and VASOTEC™ at the 10 and 15 day timepoints.

### Example 4

### Effect of Alcohol/Water Ratio on the Dispersion Time of Enalapril Maleate.

Enalapril maleate (50 grams) was added to 200 mL of liquid. The liquid ranged from 100% alcohol/ 0% water to 0% alcohol/100% water (see Table 1). The solutions were stirred at 200 rpm at room temperature using a Lightnin® Mixer (General Signal Controls, Rochester, NY) with the mixing blade 1 cm from the bottom of the beaker. Enalapril maleate was considered "dispersed" when all of the drug powder was wetted and had become immersed in the liquid.

As shown in Table 1, solutions containing high relative levels of alcohol yielded faster dispersion of enalapril maleate than did solutions containing lower relative levels of alcohol. Surprisingly, an enalapril maleate suspension containing 100% alcohol had a dispersion time of 27 seconds whereas an enalapril maleate suspension containing 100% water had a dispersion time of over 76 minutes.

**Table 1. Dispersion time of enalapril maleate increased as the proportion of water in the liquid solution increased.**

| **%Alcohol / %Water** | **Volume Alcohol / Volume Water** | **Dispersion Time** |
|---|---|---|
| 100% / 0% | 200 mL / 0 mL | 27 seconds |
| 85% / 15% | 170mL / 30mL | 28 seconds |
| 75% / 25% | 150 mL / 50 mL | 30 seconds |
| 50% / 50% | 100 mL/ 100 mL | 45 seconds |
| 25% / 75% | 50 mL/ 150 mL | 1 minute, 15 seconds |
| 0% / 100% | 0 mL/ 200 mL, | 76 minutes, 43 seconds, |

### Example 5

### Comparison of stability profiles of different formulations of quinapril hydrochloride.

The level of impurities of the formulations of stabilized quinapril hydrochloride were compared to a commercial formulation of ACCUPRIL® (Parke-Davis), referred to as "Quinapril- commercial." Formulations were stored at 60°C with 75% relative humidity to simulate extended storage. Impurity levels of the formulations were assessed at 5, 10, and 15 days by measurement of quinaprilat and quinapril-DKP formation by HPLC. Formulations were stored at 60°C with 75% relative humidity to simulate extended storage. The ACCUPRIL® formulation was placed in a 60cc HDPE bottle with a 33mm metal cap with no dessicant and no dunnage. Formulations V-VII in tablet from were placed in a 60cc HDPE bottle with a 33mm metal cap with no dessicant and no dunnage. Stability of the formulations was assessed at 5, 10, and 15 days by HPLC.

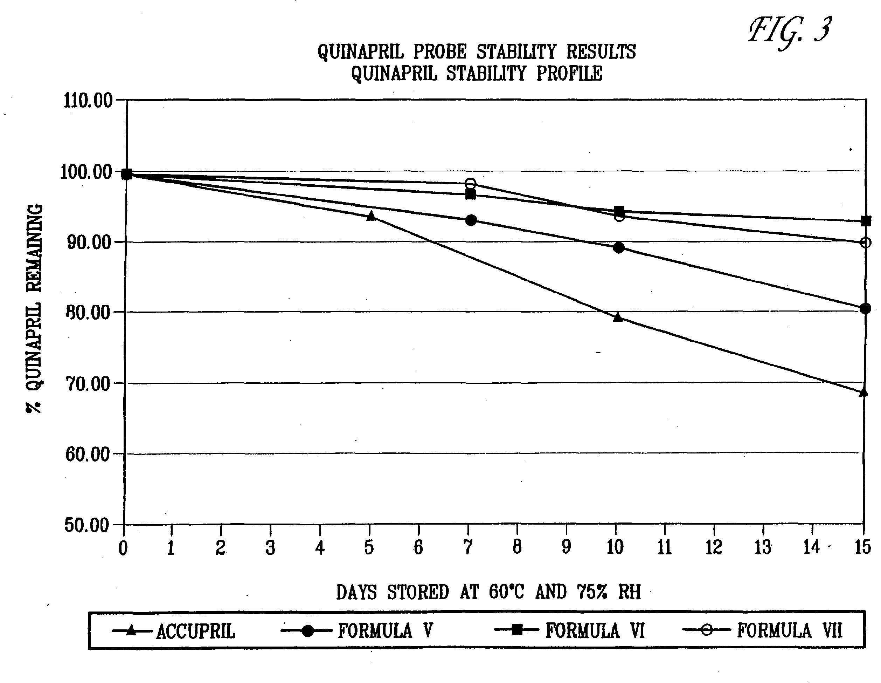

As shown in Figure 3, at the 5, 10, and 15 day timepoints, Formulations V-VII in tablet form exhibited greater purity in terms of quinaprilat content than did the ACCUPRIL® tablet.

### Example 6

### Comparison of levels of impurities in different formulations of quinapril hydrochloride.

The levels of impurities in different formulations of quinapril hydrochloride were compared. The level of impurities of the formulations of stabilized quinapril hydrochloride were also compared to a commercial formulation of quinapril HCl, ACCUPRIL® (Parke-Davis). Formulations were stored at 60°C with 75% relative humidity to simulate extended storage. Impurity levels of the formulations were assessed at 5, 10, and 15 days by measurement of quinaprilat and quinapril-DKP formation by HPLC.

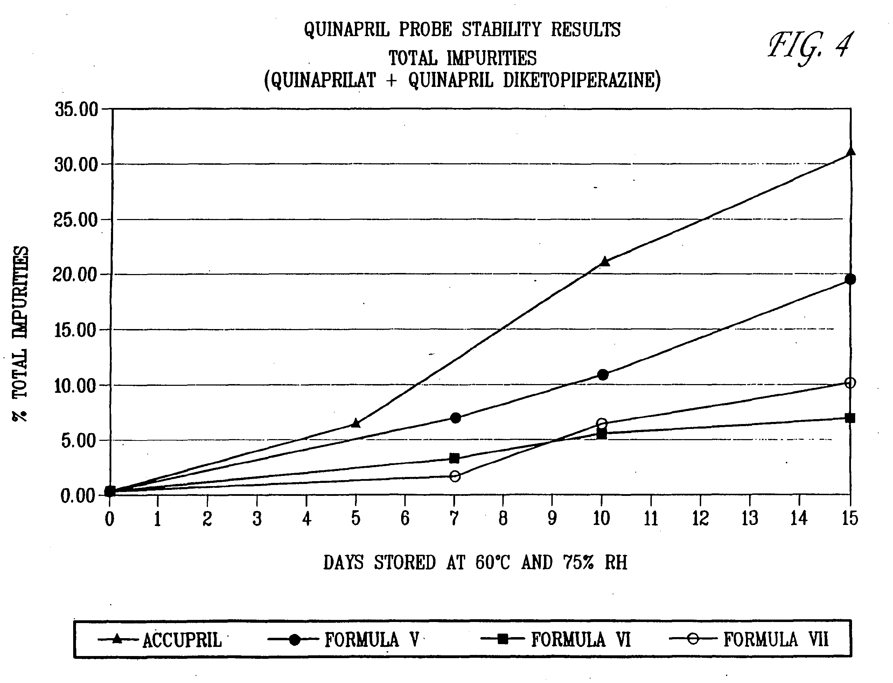

As shown in Figure 4, at all timepoints Formulations V-VII exhibited greater purity; e.g. the lowest level of impurity, than did the commercial formulation.

### Example 7

### Storage-Stability Assessment of an Industrial Scale Formulation Containing Stabilized Quinapril Hydrochloride.

The level of impurities of Formula VIII, the industrial scale tablet formulation containing stabilized quinapril hydrochloride (40 mg quinapril base per tablet), were compared to a commercial 40-mg tablet formulation of quinapril HCl, ACCUPRIL® (Parke-Davis). Tablets from both formulations were stored at accelerated stability storage conditions, namely, 40°C and 75% relative humidity to simulate extended storage. Impurity levels of the formulations were assessed at 1, 2, and 3 months by measurement of quinaprilat and quinapril-DKP formation by a validated liquid chromatography (HPLC) method. Furthermore, the levels of impurities of the same industrial scale Formula VIII obtained after storage at controlled room temperature conditions, namely, 25°C and ambient humidity (AH), were also evaluated.

As shown in Table 2, the stability profiles of Formula VIII and the commercial ACCUPRIL® formulation are comparable at all time-points of the accelerated stability study at 40°C and 75% relative humidity. Also, the controlled room temperature study clearly indicates that the stability properties of Formula VIII are superior and acceptable as per USP standards (PF) according to which, quinaprilat and quinapril-DKP are not allowed to exceed the 3% and 1% levels, respectively.

**Table 2: Impurity levels of Formula VIII and ACCUPRIL® stored at various conditions.**

| **Formulation** Impurities (by weight %) | **Initial** (0-Days) | **1 Month** 40°C/75%RH | **2 Months** 40°C/75%RH | **3 Months** 40°C/75%RH | **6 Months** 25°C/AH | **12 Months** 25°C/AH |
|---|---|---|---|---|---|---|
| **Formula VIII:** | | | | | | |
| Quinaprilat | 0.3% | 0.6% | 0.67% | 0.8% | 0.5% | 0.6% |
| Quinapril-DKP | 0.1% | 0.2% | 0.23% | 0.3% | 0.2% | 0.2% |

| **Accupril®:** | | | | | | |
|---|---|---|---|---|---|---|
| Quinaprilat | 0.2% | 0.6% | 0.77% | 0.9% | N/A | N/A |
| Quinapril-DKP | 0.15% | 0.2% | 0.27% | 0.3% | N/A | N/A |

### Example 8

### Bio-Stability Assessment of Stabilized Quinapril Hydrochloride Formulations.

The levels of the hydrolytic degradation by-product quinaprilat created from fresh and storage-stressed tablets of Formulas VIII, IX, and X and the commercial ACCUPRIL® formulation after dissolution/dispersion in water for 3 hours, were compared. Specifically, freshly prepared tablets (of 40-mg quinapril-base strength) from each formulation were placed in glass dissolution vessels (one tablet per vessel). Each glass vessel contained 900 mL of purified water maintained at a temperature of 37°C and stirred at a paddle speed of 50 rpm. After three hours, samples were withdrawn from each vessel and analyzed for their quinaprilat content. The same experiments were repeated using storage-stressed tablets from each formulation. That is, such tablets were first stored at 60°C and 75% relative humidity for 10 days, and then they were subjected to the aforementioned 3-hr dissolution experiment and analyzed for their quinaprilat content.

As shown in Table 3, the projected bio-stability of the stabilized quinapril hydrochloride Formulas VIII, IX, and X prepared according to the invention disclosed herein, is significantly superior that that of the commercial ACCUPRIL® brand tablet formulation. After being dissolved/dispersed in 37°C water for three hours, fresh and storage-stressed tablets of Formulas VIII, IX, and X demonstrated quinaprilat levels that did not exceed 0.7% by weight (for fresh tablets) and 4% by weight (for storage-stressed tablets). On the other hand, the ACCUPRIL® brand tablets suffered significantly higher levels of hydrolysis and produced 3.4% by weight of quinaprilat when fresh ACCUPRIL® tablets were used and about 11.1% by weight of quinaprilat when storage-stressed tablets were tested.

**Table 3: Quinaprilat impurity levels obtained from fresh and storage-stressed tablets of Formulas VIII, IX, and X and the commercial ACCUPRIL® brand after exposure for 3 hours to 900 mL of purified water maintained at 37°C and stirred at a paddle speed of 50 rpm.**

| **Quinaprilat Levels by weight %** | **Formula VIII** | **Formula IX** | **Formula X** | **Accupril® Brand** |
|---|---|---|---|---|
| **Quinaprilat:** | | | | |
| Obtained from | 0.58% | 0.50% | 0.65% | 3.4% |
| **Fresh Tablets Dissolved in Water** | | | | |
| **Quinaprilat:** | | | | |
| Obtained from | 3.3% | 3.2% | 4.0% | 11.1% |
| **Storage-Stressed TabletsDissolved in Water** | | | | |

Furthermore, as shown in Table 4, the Bio/Storage Stability Ratio (BSSR-value) of the stabilized quinapril hydrochloride Formula VIII prepared in industrial scale according to the invention disclosed herein, is significantly lower than that of the commercial ACCUPRIL® brand tablet formulation. The BSSR-value is the ratio of the quinaprilat levels or, in general, the levels of the hydrolytic degradation product of the ACE inhibitor, obtained from the Bio-Stability assessment using the 3-hr dissolution test discussed previously with respect to Table 3 (this quinaprilat level is placed in the numerator), and from the 3-month timepoint of the accelerated Storage-Stability study conducted at 40°C and 75% relative humidity (RH) discussed with respect to Table 2 (this quinaprilat level is placed in the denominator). As shown in Table 4, the BSSR-value of Formula VIII is only 0.73 which is significantly lower than the 3.78 BSSR-value displayed by the commercial ACCUPRIL® brand product. Therefore, according to the findings presented herein, the term "simultaneously storage-stable and bio-stable compositions of ACE inhibitors" can be defined as those formulations of ACE inhibitors which do not contain more than 5% of the hydrolytic breakdown product of the ACE inhibitor while at the same time displaying a Bio/Storage Stability Ratio lower than about 3.5 (which is lower than the BSSR-value of 3.78 obtained by the commercial ACCUPRIL® brand product).

**Table 4: Comparison of Bio/Storage Stability Ratios (BSSR) demonstrated by tablets of Quinapril Hydrochloride of Formula VIII and the commercial ACCUPRIL® brand.**

| **Formulations** | **Bio-Stabitity** Quinaprilat produced after 3-hr dissolution in 37°C water | **Storage-Stability** Quinaprilat produced after 3-month storage (40°C/75% RH) | **Bio/Storage Stability Ratio** (Ratio of Bio-stability and Storage-Stability) |
|---|---|---|---|
| **Formula VIII** Stabilized Quinapril-HCl Tablets | 0.58% | 0.8% | **0.73** |
| **ACCUPRIL®** Commercial Brand Quinapril-HCl Tablets | 3.4% | 0.9% | **3.78** |

The finding discussed with respect to Tables 3 and 4 clearly indicate that, while possessing excellent manufacturing characteristics, the stabilized quinapril hydrochloride and other ACE inhibitors formulations prepared according the invention presented herein, are not only storage-stable but also bio-stable at levels much more superior than the commercial brand products such as ACCUPRIL® tablets (Parke-Davis). It should be reiterated that, with respect to bioavailability and therapeutic effectiveness, it is very important for any ACE inhibitor such as quinapril hydrochloride not to be converted to its hydrolysis degradation product such as quinaprilat when exposed to aqueous solutions which simulate the watery environment of the gastrointestinal tract. Such undesirable conversion produces a form of the ACE inhibitor that can not be absorbed by the gastrointestinal tract, thereby depriving the patient from absorbing a portion of the active substance in his/her systemic circulation and possibly promoting therapeutic variability and inequivalence. The stabilized ACE inhibitor formulations prepared according to the present invention seem to eliminate this problem by providing systems that are manufacturable and simultaneously storage-stable and bio-stable, as they are not containing more than 5% of the hydrolysis breakdown product of the ACE inhibitor and, at the same time, demonstrating Bio/Storage Stability Ratios that are lower than 3.5.

The ACE inhibitors of the present invention are selected from the group consisting of enalapril maleate, quinapril hydrochloride, benazepril hydrochloride, moexipril hydrochloride, lisonopril hydrochloride, ramipril hydrochloride, indopril hydrochloride, and pharmaceutically acceptable salts thereof.

As used in this specification and the appended claims, the singular forms "a," "an" and "the" include plural references unless the content clearly dictates otherwise. Thus, for example, reference to "an excipient" includes a mixture of two or more excipients.

## Claims

1. A method of preparing a stable formulation of an ACE inhibitor selected from enalapril maleate, quinapril hydrochloride, benazepril hydrochloride, moexipril hydrochloride, lisonopril hydrochloride, ramipril hydrochloride, indopril hydrochloride, and pharmaceutically acceptable salts thereof, which comprises the steps of:
dispersing or dissolving a metal compound in an alcohol to form a metallic alcoholic dispersion;
mixing said ACE inhibitor into said metallic alcoholic dispersion; and
mixing until a clear solution is attained,
wherein said stable formulation of an ACE inhibitor comprises less than 11% by weight of hydrolytic breakdown product after incubation at 40°C and 75% relative humidity for 10 days and subsequent contact with water maintained at 37°C for 3 hours.

2. A method as claimed in claim 1, wherein said alcohol comprises ethanol and water.

3. A method as claimed in claim 1, wherein said ACE inhibitor is quinapril hydrochloride.

4. A method as claimed in claim 1, wherein said metal compound comprises sodium bicarbonate.

5. A method as claimed in claim 1, wherein said metal is an alkali metal.

6. A method as claimed in claim 1, wherein said metal is an alkali earth metal.

7. A method as claimed in claim 1, further comprising adding at least one excipient to said clear solution.

8. A method as claimed in claim 7, further comprising adding an antioxidant to said clear solution.

9. A method as claimed in claim 8, wherein said antioxidant is selected from the group consisting of butyl hydroxyl anisol, butyl hydroxyl toluene, maleic acid, and ascorbic acid.

10. A method as claimed in claim 7, wherein said excipient comprises microcrystalline cellulose, sodium starch glycolate, or combinations thereof.

11. A storage-stable and bio-stable formulation of ACE inhibitor selected from enalapril maleate, quinapril hydrochloride, benazepril hydrochloride, moexipril hydrochloride, lisonopril hydrochloride, ramipril hydrochloride, indopril hydrochloride, and pharmaceutically acceptable salts thereof, prepared in accordance with claim 1, comprising less that 5% by weight of hydrolytic breakdown products and having a bio/storage stability ratio of less than about 3.5.

12. A formulation as claimed in claim 11, wherein said bio/storage stability ratio is less than 2.

13. A formulation as claimed in claim 12, wherein bio/storage stability ratio is less than 1.

14. A formulation as claimed in claim 11, wherein said ACE inhibitor comprises quinapril hydrochloride.

15. A formulation as claimed in claim 11, wherein said ACE inhibitor comprises enalapril maleate, quinapril hydrochloride, benazepril hydrochloride, moexipril hydrochloride, lisonopril hydrochloride, indopril hydrochloride, or combinations thereof.

16. A formulation as claimed in claim 11, wherein said ACE inhibitor is stabilized in the presence of sodium bicarbonate.

17. A bio-stable pharmaceutical formulation of ACE inhibitor selected from enalapril maleate, quinapril hydrochloride, benazepril hydrochloride, moexipril hydrochloride, lisonopril hydrochloride, ramipril hydrochloride, indopril hydrochloride, and pharmaceutically acceptable salts thereof, prepared in accordance with claim 1 comprising less than 11% by weight of hydrolytic breakdown product after incubation at 40°C and 75% relative humidity for 10 days and subsequent contact with water maintained at 37°C for 3 hours.

18. A formulation as claimed in claim 17 comprising less than 8% by weight of hydrolytic breakdown product.

19. The formulation of claim 18 comprising less than 5% by weight of hydrolytic breakdown product.

20. A bio-stable pharmaceutical formulation of ACE inhibitor selected from enalapril maleate, quinapril hydrochloride, benazepril hydrochloride, moexipril hydrochloride, lisonopril hydrochloride, ramipril hydrochloride, indopril hydrochloride, and pharmaceutically acceptable salts thereof, prepared in accordance with claim 1, comprising less than 3.5% by weight of hydrolytic breakdown product wherein said ACE inhibitor is freshly prepared and after said freshly prepared ACE inhibitor is contacted with water maintained at 37°C for 3 hours.

21. A formulation as claimed in claim 20 comprising less than 2.5% by weight of hydrolytic breakdown product.

22. A formulation as claimed in claim 21 comprising less than 1.5% by weight of hydrolytic breakdown product.

23. A pharmaceutical preparation prepared in accordance with claim 1, comprising a pharmaceutically acceptable formulation of quinapril hydrochloride substantially free of breakdown products, wherein said breakdown products comprise quinaprilat and quinapril-DKP.

24. A pharmaceutical preparation as claimed in claim 23, which contains less than 12.5% breakdown products by weight of said formulation after incubation at 60°C and 75% relative humidity for 10 days.

25. A pharmaceutical preparation as claimed in claim 24, which contains less than 6% breakdown products by weight of said formulation after incubation at 60°C and 75% relative humidity for 10 days.

26. A pharmaceutical preparation as claimed in claim 25, which contains less than 3% breakdown products by weight of said formulation after incubation at 60°C and 75% relative humidity for 10 days.

27. A pharmaceutical preparation as claimed in claim 26, which contains less than 1.5% breakdown products by weight of said formulation after incubation at 60°C and 75% relative humidity for 10 days.

28. A pharmaceutical preparation as claimed in claim 23, wherein said preparation is freshly prepared and which contains less than 3.5% quinaprilat by weight of said formulation after contact with water maintained at 37°C for 3 hours.

29. A pharmaceutical preparation as claimed in claim 28 which contains less than 2% by weight quinaprilat.

30. A pharmaceutical preparation as claimed in claim 29 which contains less than 1.5% by weight quinaprilat.

31. A storage-stable and bio-stable formulation of ACE inhibitor selected from enalapril maleate, quinapril hydrochloride, benazepril hydrochloride, moexipril hydrochloride, lisonopril hydrochloride, ramipril hydrochloride, indopril hydrochloride, and pharmaceutically acceptable salts thereof, prepared in accordance with claim 1, comprising less than 5% by weight of hydrolytic breakdown products and having a bio/storage stability ratio of less than about 3.5, for use in a method of treating a cardiovascular disorder.

32. A method of preparing a stable formulation of quinapril hydrochloride which comprises the steps of:
dispersing or dissolving a sodium compound in an alcohol to form a metallic alcoholic dispersion;
mixing quinapril hydrochloride into said metallic alcoholic dispersion to form a metallic alcoholic and active ingredient dispersion;
mixing a thickening agent into said metallic alcoholic and active ingredient dispersion; and
mixing until a clear solution is attained, wherein said stable formulation of quinapril hydrochloride contains less than 12.5% breakdown products by weigh of said formulation after incubation at 60°C and 75% relative humidity for 10 days.

33. A method as claimed in claim 32, wherein said thickening agent comprises polyvinylpyrrolidone, polyethylene glycol, polyvinyl alcohol, or combinations thereof.

34. A method as claimed in claim 32, wherein said alcohol comprises ethanol and water.

35. A method as claimed in claim 32, wherein said sodium compound comprises sodium bicarbonate.

36. A method as claimed in claim 32, further comprising adding an antioxidant to said clear solution.

37. A method as claimed in claim 36, wherein said antioxidant is butyl hydroxyl anisol, butyl hydroxyl toluene, maleic acid, ascorbic acid, or combinations thereof.

38. A method as claimed in claim 32, further comprising adding at least one excipient to said clear solution.

39. A method as claimed in claim 38, wherein said excipient comprises microcrystalline cellulose, sodium starch glycolate, or combinations thereof.

40. A storage-stable and bio-stable pharmaceutical preparation of quinapril hydrochloride prepared in accordance with claim 32.

41. A pharmaceutical preparation as claimed in claim 40, comprising less than 5% by weight quinaprilat and having a bio/storage stability ratio of less than about 3.5.

42. A pharmaceutical preparation as claimed in claim 41, wherein said bio/storage stability ratio is less than 2.

43. A pharmaceutical preparation as claimed in claim 42 wherein said bio/storage stability ratio is less than 1.

44. A pharmaceutical preparation as claimed in claim 40 which contains less than 12.5% breakdown products by weight of said formulation after incubation at 60°C and 75% relative humidity for 10 days.

45. A pharmaceutical preparation as claimed in claim 44 which contains less than 6% breakdown products by weight of said formulation after incubation at 60°C and 75% relative humidity for 10 days.

46. A pharmaceutical preparation as claimed in claim 45 which contains less than 3% breakdown products by weight of said formulation after incubation at 60°C and 75% relative humidity for 10 days.

47. A pharmaceutical preparation as claimed in claim 46 which contains less than 1.5% breakdown products by weight of said formulation after incubation at 60°C and 75% relative humidity for 10 days.

48. A pharmaceutical preparation as claimed in claim 40 wherein said preparation is freshly prepared and which contains less than 3.5% quinaprilat by weight of said formulation after contact with water maintained at 37°C for 3 hours.

49. A pharmaceutical preparation as claimed in claim 48 which contains less than 2.5% by weight quinaprilat.

50. A pharmaceutical preparation as claimed in claim 49 which contains less than 1.5% by weight quinaprilat.

51. A pharmaceutical preparation as claimed in claim 40 comprising less than 11% by weight quinaprilat after incubation at 40°C and 75% relative humidity for 10 days and subsequent contact with water maintained at 37°C for 3 hours.

52. A pharmaceutical preparation as claimed in claim 51 comprising less than 8% by weight quinaprilat.

53. A pharmaceutical preparation as claimed in claim 52 comprising less than 5% by weight quinaprilat.

54. A storage-stable and bio-stable formulation of quinapril hydrochloride prepared in accordance with claim 32, comprising less than 5% by weight quinaprilat and having a bio/storage stability ratio of less than about 3.5, for use in a method of treating a cardiovascular disorder.

## Patentansprüche

1. Verfahren zur Herstellung einer stabilen Formulierung eines ACE-Hemmers, der aus Folgenden ausgewählt ist: Enalaprilmaleat, Quinapril-Hydrochlorid, Benazepril-Hydrochlorid, Moexipril-Hydrochlorid, Lisonopril-Hydrochlorid, Ramipril-Hydrochlorid, Indopril-Hydrochlorid und pharmazeutisch verträglichen Salzen davon, das die folgenden Schritte umfasst:
Dispergieren oder Lösen einer Metallverbindung in eine Alkohol, um eine alkoholische Dispersion zu bilden;
Mischen des genannten ACE-Hemmers in der genannten metallischen alkoholischen Dispersion; und
Mischen bis eine klare Lösung erhalten wird,
worin die genannte stabile Formulierung eines ACE-Hemmers weniger als 11 Gewichts-% an hydrolytische Abbauprodukt nach Inkubation bei 40°C und 75 % relativer Feuchtigkeit für 10 Tage und anschließendem Kontakt mit Wasser, der bei 37°C für 3 Stunden aufrechterhalten wird, umfasst.

2. Verfahren nach Anspruch 1, worin der genannte Alkohol Ethanol und Wasser umfasst.

3. Verfahren nach Anspruch 1, worin der genannte ACE-Hemmer Quinapril-Hydrochlorid ist.

4. Verfahren nach Anspruch 1, worin die genannte Metallverbindung Natriumbicarbonat umfasst.

5. Verfahren nach Anspruch 1, worin das genannte Metall ein Alkalimetall ist.

6. Verfahren nach Anspruch 1, worin das genannte Metall ein Erdalkalimetall ist.

7. Verfahren nach Anspruch 1, das weiter das Zugeben von mindestens einem Hilfsstoff zu der genannten klaren Lösung umfasst.

8. Verfahren nach Anspruch 7, das weiter das Zugeben eines Antioxidationsmittels zu der genannten klaren Lösung umfasst.

9. Verfahren nach Anspruch 8, worin das genannte Antioxidationsmittel aus der Gruppe ausgewählt ist, die aus Butylhydroxylanisol, utylhydroxyltoluol, Maleinsäure und Ascorbinsäure besteht.

10. Verfahren nach Anspruch 7, worin der genannte Hilfsstoff mikrokristalline Cellulose, Natrium-Stärke-Glycolat kombinationen davon umfasst.

11. Lagerstabile und biologisch stabile Formulierung eines ACE-Hemmers, der aus Folgenden ausgewählt ist: Enalaprilmaleat, Quinapril-Hydrochlorid, Benazepril-Hydrochlorid, Moexipril-Hydrochlorid, Lisonopril-Hydrochlorid, Ramipril-Hydrochlorid, Indopril-Hydrochlorid und pharmazeutisch verträglichen Salzen davon, die gemäß Anspruch 1 hergestellt wird, die weniger als 5 Gewichts-% an hydrolytischen Abbauprodukten umfasst und ein Verhältnis von biologischer Stabilität/Lagerstabilität von kleiner als etwa 3,5 aufweist.

12. Formulierung anspruch 11, worin das Verhältnis von biologischer Stabilität/Lagerstabilität kleiner als 2 ist.

13. Formulierung nach Anspruch 12, worin das Verhältnis von biologischer Stabilität/Lagerstabilität kleiner als 1 ist.

14. Formulierung nach Anspruch 11, worin der genannte ACE-Hemmer Quinapril-Hydrochlorid umfasst.

15. Formulierung nach Anspruch 11, worin der genannte ACE-Hemmer Enalaprilmaleat, Quinapril-Hydrochlorid, Benazepril-Hydrochlorid, Moexipril-Hydrochlorid, Lisonopril-Hydrochlorid, Indopril-Hydrochlorid oder Kombinationen davon umfasst.

16. Formulierung nach Anspruch 11, worin der genannte ACE-Hemmer in Gegenwart von Natriumbicarbonat stabilisiert wird.

17. Biologisch stabile pharmazeutische Formulierung eines ACE-Hemmers, der aus Folgenden ausgewählt ist: Enalaprilmaleat, Quinapril-Hydrochlorid, Benazepril-Hydrochlorid, Moexipril-Hydrochlorid, Lisonopril-Hydrochlorid, Ramipril-Hydrochlorid, Indopril-Hydrochlorid und pharmazeutisch verträglichen Salzen davon, die gemäß Anspruch 1 hergestellt wird, die weniger als 11 Gewichts-% an hydrolytischem Abbauprodukt nach Inkubation bei 40°C und 75 % relativer Feuchtigkeit für 10 Tage und anschließendem mit Wasser, der bei 37°C für 3 Stunden aufrechterhalten wird, umfasst.

18. Formulierung nach Anspruch 17, die weniger als 8 Gewichts-% an hydrolytischem Abbauprodukt umfasst.

19. Formulierung nach Anspruch 18, die weniger als 5 Gewichts-% an hydrolytischen Abbauprodukt umfasst.

20. Biologisch stabile pharmazeutische Formulierung eines ACE-Hemmers, der aus Folgenden ausgewählt ist: Enalaprilmaleat, Quinapril-Hydrochlorid, Benazepril-Hydrochlorid, Moexipril-Hydrochlorid, Lisonopril-Hydrochlorid, RamiprilHydrochlorid, Indopril-Hydrochlorid und pharmazeutisch verträglichen Salzen davon, die gemäß Anspruch 1 hergestellt wird, die weniger als 3,5 Gewichts-% an hydrolytischen Abbauprodukt, worin der genannte ACE-Hemmer frisch hergestellt wird und anschließend der genannte frisch hergestellte ACE-Hemmer mit Wasser in Kontakt gebracht wird, der bei 37°C für 3 Stunden aufrechterhalten wird, umfasst.

21. Formulierung Anspruch 20, die weniger als 2,5 Gewichts-% an hydrolytische Abbauprodukt umfasst.

22. Formulierung nach Anspruch 21, die weniger als 1,5 Gewichts-% an hydrolytischem Abbauprodukt umfasst.

23. Pharmazeutisches Präparat, das gemäß Anspruch 1 hergestellt wird, das eine pharmazeutisch verträgliche Formulierung von Quinapril-Hydrochlorid umfasst, die im Wesentlichen frei von Abbauprodukten ist, worin die genannten Abbauprodukte Quinaprilat und Quinapril-DKP umfassen.

24. Pharmazeutisches Präparat nach Anspruch 23, das weniger als 12,5 Gewichts-% an Abbauprodukten der Formulierung Inkubation bei 60°C und 75 % relativer Feuchtigkeit für 10 Tage enthält.

25. Pharmazeutisches Präparat nach Anspruch 24, das weniger als 6 Gewichts-% an Abbauprodukten der genannten Formulierung nach Inkubation bei 60°C und 75 % relativer Feuchtigkeit für 10 Tage enthält.

26. Pharmazeutisches Präparat nach Anspruch 25, das weniger als 3 Gewichts-% an Abbauprodukten der genannten Formulierung nach Inkubation bei 60°C und 75 % relativer Feuchtigkeit für 10 Tage enthält.

27. Pharmazeutisches Präparat nach Anspruch 26, das weniger als 1,5 Gewichts-% an Abbauprodukten der Formulierung nach Inkubation bei 60°C und 75 % relativer Feuchtigkeit für 10 Tage enthält.

28. Pharmazeutisches Präparat nach Anspruch 23, worin das genannte Präparat frisch hergestellt wird und das weniger als 3,5 Gewichts-% an Quinaprilat der genannten Formulierung nach Kontakt mit Wasser, der bei 37°C für 3 Stunden aufrechterhalten wird, enthält.

29. Pharmazeutisches Präparat nach Anspruch 28, das weniger als 2 Gewichts-% an Quinaprilat enthält.

30. Pharmazeutisches nach Anspruch 29, das weniger als 1,5 Gewichts-% an Quinaprilat enthält.

31. Lagerstabile und biologisch Formulierung eines ACE-Hemmers, der aus Folgenden ausgewählt ist: Enalaprilmaleat, Quinapril-Hydrochlorid, BenazeprilHydrochlorid, Moexipril-Hydrochlorid, Lisonopril-Hydrochlorid, Ramipril-Hydrochlorid, Indopril-Hydrochlorid und pharmazeutisch verträglichen Salzen davon, die gemäß Anspruch 1 hergestellt wird, die weniger als 5 Gewichts-% an hydrolytischen Abbauprodukten umfasst und ein Verhältnis von biologischer Stabilität/Lagerstabilität von kleiner als etwa 3,5 aufweist, zur Verwendung in einem Verfahren zur Behandlung einer kardiovaskulären Erkrankung.

32. Verfahren zur Herstellung einer Formulierung von Quinapril-Hydrochlorid, das die Schritte umfasst:
Dispergieren oder Lösen einer Natriumverbindung in eine Alkohol, um eine alkoholische Dispersion zu bilden;
Mischen von Quinapril-Hydrochlorid in der genannten alkoholisches Dispersion, um eine metallische alkoholische und Wirkstoff-Dispersion zu bilden;
Mischen eines Verdickungsmittels in die metallische alkoholische und Wirkstoff-Dispersion; und
Mischen bis eine Lösung erhalten wird, worin die genannte stabile Formulierung von Quinapril-Hydrochlorid als 12,5 Gewichtes-% an Abbauprodukten der Formulierung nach Inkubation bei 60°C und 75 % relativer Feuchtigkeit für 10 Tage enthält.

33. Verfahren nach Anspruch 32, worin das genannte Verdickungsmittel Polyvinylpyrrolidon, Polyethylenglycol, Polyvinylalkohol kombinationen davon umfasst.

34. Verfahren nach Anspruch 32, worin der genannte Alkohol und Wasser umfasst.

35. Verfahren nach Anspruch 32, worin die genannte Natriumverbindung Natriumbicarbonat umfasst.

36. Verfahren nach Anspruch 32, das weiter das Zugeben eines Antioxidationsmittels zu der genannten klaren Lösung umfasst.

37. Verfahren nach Anspruch 36, worin das genannte Antioxidationsmittel Butylhydroxylanisol, utylhydroxyltoluol, Maleinsäure, Ascorbinsäure oder Kombinationen davon ist.

38. Verfahren nach Anspruch 32, das weiter as Zugeben von mindestens einem Hilfsstoff zu der genannten Lösung umfasst.

39. Verfahren nach Anspruch 38, worin der genannte Hilfsstoff mikrokristalline Cellulose, Natrium-Stärke-Glycolat oder Kombinationen davon umfasst.

40. Lagerstabiles und biologisch stabiles pharmazeutisches Präparat von Quinapril-Hydrochlorid, das gemäß Anspruch 32 hergestellt wird.

41. Pharmazeutisches Präparat nach Anspruch 40, das weniger als 5 Gewichts-% an Quinaprilat und ein Verhältnis von biologischer Stabilität/Lagerstabilität von kleiner als etwa 3,5 aufweist.

42. Pharmazeutisches Präparat nach Anspruch 41, worin das genannte Verhältnis von biologischer Stabilität/Lagerstabilität kleiner als 2 ist.

43. Pharmazeutisches Präparat nach Anspruch 42, worin das genannte Verhältnis von biologischer Stabilität/Lagerstabilität kleiner als 1 ist.

44. Pharmazeutisches Präparat nach Anspruch 40, das weniger als 12,5 Gewichts-% an Abbauprodukten der genannten Formulierung nach Inkubation bei 60°C und 75 % relativer Feuchtigkeit für 10 Tage enthält.

45. Pharmazeutisches Präparat nach Anspruch 44, das weniger als 6 Gewichtes-% an Abbauprodukten der Formulierung nach Inkubation bei 60°C und 75 % relativer Feuchtigkeit für 10 Tage enthält.

46. Pharmazeutisches Präparat nach Anspruch 45, das weniger als 3 Gewichtes-% an Abbauprodukten der genannten Formulierung nach Inkubation bei 60°C und 75 % relativer Feuchtigkeit für 10 Tage enthält.

47. Pharmazeutisches Präparat nach Anspruch 46, das als 1,5 Gewichtes-% an Abbauprodukten der Formulierung inkubation bei 60°C und 75 % relativer Feuchtigkeit für 10 Tage enthält.

48. Pharmazeutisches Präparat nach Anspruch 40, worin das genannte frisch hergestellt wird und das weniger als 3,5 Gewichts-% an Quinaprilat der genannten Formulierung nach Kontakt mit Wasser, der bei 37°C für 3 Stunden aufrechterhalten wird, enthält.

49. Pharmazeutisches Präparat nach Anspruch 48, das weniger als 2,5 Gewichtes-% an Quinaprilat enthält.

50. Pharmazeutisches Präparat nach Anspruch 49, das weniger als 1,5 Gewichtes-% an Quinaprilat enthält.

51. Pharmazeutisches Präparat nach Anspruch 40, das weniger als 11 Gewichts-% an Quinaprilat nach Inkubation bei 40°C und 75 % relativer Feuchtigkeit für 10 Tage und anschließendem Kontakt mit Wasser, der bei 37°C für 3 Stunden aufrechterhalten wird, umfasst.

52. Pharmazeutisches Präparat nach Anspruch 51, das weniger als 8 Gewichts-% an Quinaprilat umfasst.

53. Pharmazeutisches Präparat nach Anspruch 52, das weniger als 5 Gewichtes-% an Quinaprilat umfasst.

54. Lagerstabile und biologisch Formulierung von Quinapril-Hydrochlorid, die gemäß Anspruch 32 hergestellt wird, die weniger als 5 Gewichts-% an Quinaprilat umfasst und ein Verhältnis von biologischer Stabilität/Lagerstabilität von kleiner als etwa 3,5 aufweist, zur Verwendung in eine Verfahren zur Behandlung einer kardiovaskulären Erkrankung.

## Revendications

1. Méthode de préparation d'une formulations stable d'un inhibiteur ACE choisi te maléate d'énalapril, le chlorhydrate de quinapril, le chlorhydrate de bénazéprit, le chlorhydrate de moéxipril, le chlorhydrate de lisinopril, le chlorhydrate de ramipril, le chlorhydrate d'indopril, et des sels pharmaceutiquement acceptables de ceux-ci, comprenant les étapes consistant à :
disperser ou solubiliser un compose de métal dans un alcool afin de former une alcoolique métallique ;
mélanger ledit inhibiteur ACE dans dispersion alcoolique métallique ; et
mélanger jusqu'à obtention d'une solution limpide ;
ou formulation stable d'un inhibiteur ACE comprend moins de 11% en poids de produit de dégradation hydrolytique après incubation à 40°C et 75% d'humidité relative pendant 10 jours puis contact avec de l'eau maintenue à 37°C pendant 3 heures.

2. Méthode selon la revendication 1, dans laquelle ledit alcool comprend de l'éthanol et de l'eau.

3. Méthode selon la revendication 1, dans laquelle ledit inhibiteur ACE est le chlorhydrate de quinapril.

4. Méthode selon la revendication 1, dans laquelle ledit composé de métal comprend du bicarbonate de sodium.

5. Méthode selon la revendication 1, dans laquelle ledit métal est un métal alcalin.

6. Méthode selon la revendication 1, dans laquelle ledit est un métal alcalino-terreux.

7. Méthode selon la revendication 1, comprenant en outre l'addition d'au moins un excipient à ladite solution limpide.

8. Méthode selon la revendication 7, comprenant en outre l'addition d'un antioxydant à ladite solution limpide.

9. Méthode selon la revendication 8, dans laquelle ledit antioxydant est choisi dans le groupe constitué par le butylhydroxy-anisol, le butylhydroxytoluène, l'acide maléique et l'acide ascorbique.

10. Méthode selon la revendication 7, dans laquelle ledit excipient comprend de la cellulose microcristalline, du glycolate d'amidon sodique, ou des combinaisons de ceux-ci.

11. Formulation stable au stockage et biostable d'inhibiteur ACE choisi parmi le maléate d'énalapril, le chlorhydrate de quinapril, le chlorhydrate de bénazépril, le chlorhydrate de moéxipril, le chlorhydrate de lisinopril, le chlorhydrate de ramipril, le chlorhydrate d'indopril, et des sels pharmaceutiquement acceptables de ceux-ci, conformément à la revendication 1, comprenant moins de 5% en poids de produits de dégradation hydrolytique et ayant un rapport de biostabilité/stabilité au stockage inférieur à environ 3,5.

12. Formulation selon la revendication 11, dans laquelle ledit rapport de biostabilité/stabilité au stockage est inférieur à 2.

13. Formulation selon la revendication 12, dans laquelle le rapport de biostabilité/stabilité au stockage est inférieur à 1.

14. Formulation selon la revendication 11, dans laquelle ledit inhibiteur ACE comprend du chlorhydrate de quinapril.

15. Formulation selon la revendication 11, dans laquelle ledit inhibiteur ACE comprend du maléate d'énalapril, du chlorhydrate de quinapril, du chlorhydrate de bénazépril, du chlorhydrate de moéxipril, du chlorhydrate de lisinopril, du chlorhydrate d'indopril, ou des combinaisons de ceux-ci.

16. Permutation selon la revendication 11, dans laquelle inhibiteur ACE est stabilisé en présence de bicarbonate de sodium.

17. Formulation pharmaceutique biostable d'inhibiteur ACE choisi pami le maléate d'énalapril, le chlorhydrate de quinapril, le chlorhydrate de bénazépril, le chlorhydrate de moéxipril, le chlorhydrate de lisinopril, le chlorhydrate de ramipril, le chlorhydrate d'indopril, et des sels pharmaceutiquement acceptables de ceux-ci, préparée conformément à la revendication 1, comprenant moins de 11% en poids de produit de dégradation hydrolytique incubation à 40°C et 75% d'humidité relative pendant 10 jours puis contact avec de l'eau maintenue à 37°C pendant 3 heures.

18. Formulations selon la revendication 17, comprenant moins de 8% en poids de produit de dégradatian hydrolytique.

19. Formulations selon la revendication 18, comprenant moins de 5% en poids de produit de dégradation hydrolytique.

20. Formulation pharmaceutique d'inhibiteur ACE choisi parmi le maléate d'énalapril, le chlorhydrate de quinapril, le chlorhydrate de bénazépril, le chlorhydrate de moéxipril, le chlorhydrate de lisinopril, le chlorhydrate de ramipril, le chlorhydrate d'indopril, et des pharmaceutiquement acceptables de ceux-ci, préparée conformément à la revendication 1, comprenant de 3,5% en poids de produit de hydrolylique, où inhibiteur ACE est fraîchement prépare puis ledit inhibiteur ACE fraîchement est mis en contact avec de l'eau maintenue à 37°C pendant 3 heures.

21. Formulations selon la revendication 20, comprenant moins de 2,5% en poids de produit de dégradatian hydrolytique.

22. Formulation selon la revendication 21, comprenant moins de 1,5% en poids de produit de dégradation hydrolytique.

23. Préparation pharmaceutique conformément à la revendication 1, comprenant une formulation pharmaceutiquement acceptable de chlorhydrate de quinapril sensiblement dépourvue de produits de dégradation, où lesdits produits de comprennent du et du quinapril-DKP.

24. Préparation pharmaceutique selon la revendication 23, contenant moins de 12,5% de produits de dégradation en poids de formulation après incubation à 60°C et 75% d'humidité relative pendant 10 jours.

25. Préparation pharmaceutique selon la revendication 24, contenant moins de 6% de produits de dégradation en poids de formulation incubation à 60°C et 75% d'humidité relative pendant 10 jours.

26. Préparation pharmaceutique selon la revendication 25, contenant moins de 3% de produits de dégradation en poids de formulation après incubation à 60°C et 75% d'humidité relative pendant 10 jours.

27. Préparation pharmaceutique selon la revendication 26, contenant moins de 1,5% de produits de en poids de formulation après incubation à 60°C et 75% d'humidité relative pendant 10 jours.

28. Préparation pharmaceutique selon la revendication 23, dans laquelle ladite préparation est fraîchement préparée et contient moins de 3,5% de quinaprilate en poids s de formulation après contact avec de l'eau maintenue à 37°C pendant 3 heures.

29. Préparation pharmaceutique selon la revendication 2 , contenant moins de 2% en poids de quinaprilate.

30. Préparation pharmaceutique selon la revendication 29, contenant moins de 1,5% en poids de quinaprilate.

31. Formulation stable au stockage et biostable d'inhibiteur ACE choisi parmi le maléate d'énalapril, le chlorhydrate de quinapril, le chlorhydrate de bénazépril, le chlorhydrate de moéxipril, le chlorhydrate de lisinopril, le chlorhydrate de ramipril, le chlorhydrate d'indopril, et des sels pharmaceutiquement acceptables ceux-ci, conformément à la revendication 1, comprenant moins de 5% en poids de produits de dégradation hydrolytique et ayant un rapport de biostabilité/stabilité au stockage inférieur à environ 3,5, pour une utilisation dans une méthode de traitement d'un trouble cardiovasculaire.

32. Méthode de préparation d'une formulation stable de chlorhydrate de quinapril, comprenant les étapes consistant à :
disperser ou solubiliser un composé de sodium dans un alcool afin de former une alcoolique métallique ;
mélanger le chlorhydrate de quinapril dans ladite dispersion alcoolique métallique afin de former une alcoolique métallique et d'ingrédient actif ;
mélanger un agent épaississant dans ladite dispersion alcoolique métallique est d'ingrédient actif ; et
mélanger jusqu'à obtention d'une solution limpide ;
où formulation stable de chlorhydrate de quinapril comprend moins de 12,5% de produit de dégradation en poids de formulation après incubation à 60°C et 75% d'humidité relative pondant 10 jours.

33. Méthode selon la revendication 32, dans laquelle ledit agent épaississant comprend de la polyvinylpyrrolidone, du polyéthylène glycol, de l'alcool polyvinylique, ou des combinaisons de ceux-ci.

34. Méthode selon la revendication 32, dans laquelle ledit alcool comprend de l'éthanol et de l'eau.

35. Méthode selon la revendication 32, dans laquelle ledit composé de sodium comprend du bicarbonate de sodium.

36. Méthode selon la revendication 32, comprenant en outre l'addition d'un antioxydant à ladite solution limpide.

37. Méthode selon la revendication 36, dans laquelle ledit est le butylhydroxy-anisol, le butylhydroxytoluène, l'acide maléique, l'acide ascorbique, ou des combinaisons de ceux-ci.

38. Méthode selon la revendication 32, comprenant en outre l'addition d'au moins un excipient à ladite solution limpide.

39. Méthode selon la revendication 38, laquelle excipient comprend de la cellulose microcristalline, du glycolate d'amidon sodique, ou des combinaisons de ceux-ci.

40. Préparation pharmaceutique stable au stockage et biostable de chlorhydrate de quinapril, préparée conformément à la revendication 32.

41. Préparation pharmaceutique selon la revendication 40, comprenant moins de 5% en poids de quinaprilate et ayant un rapport de biostabilité/stabilité au stockage inférieur à environ 3,5.

42. Préparation pharmaceutique selon la revendication 41, dans laquelle ledit rapport de biostabilité/stabilité au stockage est inférieur à 2.

43. Préparation pharmaceutique selon la revendication 42, dans laquelle ledit rapport de biostabilité/stabilité au stockage est inférieur à 1.

44. Préparation pharmaceutique selon la revendication 40, contenant moins de 12,5% de produits de en poids de formulation après incubation à 60°C et 75% d'humidité relative pendant 10 jours.

45. Préparation pharmaceutique selon la revendication contenait moins de 6% de produits de dégradation en poids de formulation aprés incubation à 60°C et 75% d'humidité relative pendant 10 jours.

46. pharmaceutique selon la revendication 45, contenant moins de 3% de produits de dégradation en poids de ladite formulation incubation à 60°C et 75% d'humidité relative pendant 10 jours.

47. Préparation pharmaceutique selon la revendication 46, contenant moins de 1,5% de produits de dégradation en poids de ladite formulation après incubation à 60°C et 75% d'humidité relative pendant 10 jours.

48. pharmaceutique selon la revendication 40, dans laquelle ladite préparation est fraîchement préparée et moins de 3,5% de en poids de formulation après contact avec de l'eau à 37°C pendant 3 heures.

49. pharmaceutique selon la revendication 48, contenant moins de 2,5% en poids de quinaprilate.

50. Préparation pharmaceutique selon la revendication 49, contenant moins de 1,5% en poids de quinaprilate.

51. Préparation pharmaceutique selon la revendication 40, contenant moins de 11% en poids de quinaprilate incubation à 40°C et 75% d'humidité relative pendant 10 jours puis contact avec de l'eau maintenue à 37°C pendant 3 heures.

52. Préparation pharmaceutique selon la revendication 51, contenant moins de 8% en poids de quinaprilate.

53. Préparation pharmaceutique selon la revendication 52, contenant moins de 5% en poids de quinaprilate.

54. Formulation stable au est biostable de chlorhydrate de quinapril, préparée conformément à la revendication 32, comprenant moins de 5% en poids de quinaprilate et ayant un rapport de biostabilité/stabilité au stockage inférieur à environ 3,5, pour une utilisation dans une méthode de traitement d'un trouble cardiovasculaire.
